Europäisches Patentamt

European Patent Office

Office européen des brevets

(11) Publication number: **0 614 973 A1**

## EUROPEAN PATENT APPLICATION

(21) Application number: **94102082.8**

(22) Date of filing: **07.03.88**

(51) Int. Cl.5: **C12N 15/00**, G01N 33/569, C07K 15/00

The microorganism(s) has (have) been deposited with ATCC....... under number(s) 40270...

(30) Priority: **06.03.87 US 23113**

(43) Date of publication of application: **14.09.94 Bulletin 94/37**

(60) Publication number of the earlier application in accordance with Art.76 EPC: **0 373 168**

(84) Designated Contracting States: **AT BE CH DE FR GB IT LI LU NL SE**

(71) Applicant: **SYNERGEN INCORPORATED 1885 33rd Street Boulder, CO 80303 (US)**

(72) Inventor: **McDonell, Michael 4264 Redwood Place Boulder, Co. 80301 (US)** Inventor: **Cox, George N. 678 West Willow Street Louisville, Co. 80027 (US)**

(74) Representative: **Patentanwälte Grünecker, Kinkeldey, Stockmair & Partner Maximilianstrasse 58 D-80538 München (DE)**

(54) **Polypeptide useful in diagnosis of coccidia infection and recombinant-DNA methods for manufacturing same.**

(57) A class of polypeptides useful in an in vitro diagnosis of coccidiosis infection in chickens is disclosed. These polypeptides are also capable of inducing an immune response in chickens which were previously not exposed to Eimeria. Recombinant DNA methods for the production of these polypeptides and phage vectors useful in these methods are also disclosed.

EP 0 614 973 A1

Rank Xerox (UK) Business Services
(3.10/3.09/3.3.4)

The present invention relates to a class of polypeptides useful in diagnostic assays to determine the presence of antibodies to coccidia organisms in mammals and fowl and particularly in chickens. The invention also relates to recombinant DNA methods for the manufacture of these polypeptides and recombinant phage clones containing DNA sequences suitable for use in the recombinant methods.

Coccidiosis in chickens is caused by a variety of Eimeria species. Eimeria is endemic in chicken flocks throughout the world with the U.S.D.A. estimating in 1965 that the loss to poultry farmers in the U.S. due to Eimeria infections was in excess of 35 million dollars.

While the various Eimeria species generally have the same life cycle, the site of invasion of gastro-intestinal epithelial cells by these organisms varies according to species. Normally, E. tenella is found only in the cecae of the chicken. E. necatrix inhabits the intestine while E. mivati and E. brunetti normally parasitize the posterior intestinal tissues. E. acervulina and E. maxima tend to invade the duodenum.

By way of example of the life cycle of Eimeria, an Eimeria tenella infection begins when a chicken ingests food or water that is contaminated with sporulated oocysts. The oocyst wall ruptures in the bird's gizzard and the activated sporozoites leave the sporocyst in the small intestine. Once in the cecae, the sporozoites enter the cells of the surface epithelium, pass through the basement membrane into the lamina propria and locate in glands of Lieberkuhn. The sporozoites invade the glandular epithelial cells of the crypt, where they locate between the cell nucleus and the plasma membrane.

Within the glandular epithelial cell, the sporozoite becomes a trophozoite feeding on the host cell and enlarging to become a schizont. During schizogony, the schizont divides into about 900 first-generation merozoites. The first-generation merozoites break out into the lumen of the cecae about three days after infection, destroying the host cell. Each first-generation merozoite then enters another cecal epithelial cell and develops into a second-generation schizont.

About 300 second-generation merozoites are formed by the second schizogony. These second-generation merozoites rupture the host cell and proceed into the lumen of the cecum about 5 days after infection. Some of the second-generation merozoites enter new cells to begin a third generation of schizogony that yields up to 30 third-generation merozoites. The remainder of the second-generation merozoites enter new epithelial cells and begin gametogenesis. Fertilized oocysts can be found in the feces about 8 days after infection.

The new oocyst contains a single cell, the sporont. Development of the sporont into sporocysts and sporozoites is triggered by the presence of atmospheric oxygen and occurs outside of the chicken. The number of oocysts produced during an infection is quite large. A single oocyst containing eight sporozoites, when ingested by a chicken, can give rise to 2,000,000 second-generation merozoites.

Growth of the Eimeria in the gastro-intestinal tissues results in considerable tissue destruction, a high mortality rate in young birds and decreased weight gain in more mature animals. Release of merozoites and gametes from an intestinal cell destroys the cell. The cumulative effect of a coccidia infection may therefore be bloody diarrhea and sloughing of patches of epithelium which can lead to death. A mass of clotted blood may also form from the extensive hemorrhaging and plug the cecal opening causing destruction of the cecum. Birds that are not killed by initial infection often fail to gain weight and are susceptible to other diseases.

Because of the seriousness of an Eimeria infection, diagnostic testing methods have been sought which will indicate the presence of an infection caused by Eimeria in chickens. The present inventors have discovered a class of polypeptides useful in the diagnosis of coccidia infections. These polypeptides, when used in in vitro diagnostic assays, indicate the presence of antibodies against Eimeria organisms in infected chicken sera.

To facilitate use of these polypeptides, the present invention also relates to recombinant DNA methods for manufacturing the polypeptides. These recombinant DNA methods utilize DNA sequences contained in various recombinant phage clones which are described herein. In addition, some of these phage clones are on deposit at the American Type Culture Collection in Rockville, Maryland.

## SUMMARY OF THE INVENTION

It is an object of the present invention to provide polypeptides useful in the diagnosis of coccidial infections in chickens. It is also an object of the present invention to identify recombinant-DNA methods for the manufacture of these polypeptides.

Additional objects and advantages of the present invention will be set forth in part in the description which follows, or may be learned from the practice of the invention. The objects and advantages may be realized and attained by means of the instrumentalities and combinations particularly pointed out in the appended claims.

To achieve the objects and in accordance with the purposes of the present invention, Proteins A, B, C, D, E, F, G, H, I, J, K, L, and M have been disclosed. The DNA corresponding to these proteins is contained in various lambda phages which also are identified herein.

In addition, a recombinant-DNA method for the manufacture of polypeptides analogous to Eimeria polypeptides is disclosed. These proteins are capable of creating an immuno-diagnostic complex when exposed to sera from chickens infected with Eimeria tenella. This method comprises:

(a) Preparation of a DNA sequence capable of directing a host microorganism to produce a polypeptide possessing antigenic properties analogous to those possessed by a polypeptide produced by Eimeria organisms;

(b) Cloning the DNA sequence into a vector capable of being transferred into and replicating in a host organism, such vector containing operational elements for the DNA sequence;

(c) Transferring the vector containing the DNA sequence and operational elements into a host microorganism capable of expressing the antigenic polypeptide;

(d) Culturing the host microorganism under conditions appropriate for amplification of the vector and expression of the polypeptide; and

(e) In either order:

(i) harvesting the polypeptides; and

(ii) causing the polypeptide to assume a structure whereby it possesses antigenic properties analogous to properties possessed by polypeptides produced by Eimeria organisms.

It is understood that the foregoing general description and the following detailed description are exemplary and explanatory only and are not restrictive of the invention as claimed. The accompanying drawings, which are incorporated in and constitute a part of this specification, illustrate one embodiment of the invention and, together with the description, serve to explain the principles of the invention.

BRIEF DESCRIPTION OF THE DRAWINGS

Fig. 1    is a restriction map of phage lambda ET::Rbl-8. The region corresponding to a cDNA clone Rbl-8 is indicated.

Fig. 2    is a restriction map of phage lambda ET::Rbl-17. The region corresponding to cDNA clone Rbl-17 is indicated.

Fig. 3    is a restriction map of phage lambda ET::CH2-13. The region corresponding to cDNA clone CH2-13 is indicated.

Fig. 4    is a restriction map of phage lambda ET::CH2-15. The region corresponding to cDNA clone CH2-15 are indicated.

Fig. 5    is a restriction map of phage lambda ET:: Rbl-145. The region corresponding to cDNA clone Rbl-145 are indicated.

Fig. 6    is a restriction map of phage lambda ET::CHAD-19. The region corresponding to cDNA clone CHAD-19 is indicated.

Fig. 7    is a restriction map of phage lambda ET::Cal-3gn. The region corresponding to cDNA clone Cal-3gn is indicated.

Fig. 8    is a restriction map of phage lambda ET::Cal-4cn. The region corresponding to cDNA clone Cal-4cn is indicated.

Fig. 9    is a restriction map of phage lambda ET::Rbl-24. The region corresponding to cDNA clone Rbl-24 is indicated.

DETAILED DESCRIPTION OF THE PREFERRED EMBODIMENTS

Reference will now be made in detail to the presently preferred embodiments of the invention which, together with the drawings and the following examples, serve to explain the principles of the invention.

As noted above, the present invention relates to a class of polypeptides which are useful, inter alia, for in vitro diagnosis of coccidia infection in chickens. These substantially purified proteins are analogous to various Eimeria tenella proteins which are believed to be capable of inducing an immune response when present in chicken tissue. Because an immune response has been mounted in infected chickens against analogous antigens, the sera of such infected chickens will contain antibodies which recognize one or more of the polypeptides of the present invention. Thus, these polypeptides may serve, either in combination or individually, as the active ingredient in an in vitro diagnostic assay to determine the presence in chicken sera of antibodies directed toward various Eimeria species.

As used herein, the term "analogous," when used in connection with a protein, antigen or polypeptide, is intended to mean a polypeptide which is capable of detecting antibodies raised in response to an infection with natural Eimeria proteins in chickens. A polypeptide possessing analogous antigenic properties will thus exhibit some homology to the native Eimeria protein. It should be noted that "analogous" polypeptides, as the term is used herein, may raise an immune response which is stronger than, the same as, or weaker than the immune response raised by natural Eimeria proteins.

The following proteins, in substantially pure form, have been discovered by the present inventors as being useful in such in vitro diagnostics. These include: Protein A, a 120kd protein of E. tenella; Protein B, a 100kd protein of E. tenella; Protein C, a 200kd protein of E. tenella; Protein D, an 80kd protein of E. tenella; Protein E, an 80kd protein of E. tenella; Protein F, a 45kd protein of E. tenella; Protein G, a 30kd protein of E. tenella; Protein H, a 25kd protein of E. tenella; Protein I, a 14kd protein of E. tenella; Protein J, a 10kd protein of E. tenella; Protein K of E. tenella; Protein L of E. tenella; and Protein M of E. tenella. It should be noted that the molecular weights associated with the proteins disclosed herein are not to be interpreted as absolute values. It is intended that these values be interpreted in light of the standard deviation of ±10%.

It is believed that some of the Proteins A through M are present on the surface of the Eimeria organism at some stage in its life cycle. In particular, Protein B is believed to be present on the anterior end, in the region of the conoid, during the sporozoite stage of the organism.

Protein B appears late during the sporulation of the Eimeria and is believed to be actively synthesized by the sporulating oocyst. Protein B also cross-reacts with sera produced by animals presented with the merozoite and schizont stages, suggesting that the protein is synthesized during these stages as well. Protein B first appears approximately 44 hours into sporulation of the oocyst and persists into the sporozoite stage.

Protein A is also capable of cross-reacting with sera produced in response to either the sporozoite, merozoite or schizont stages. Protein A is thus believed to be actively synthesized and most abundantly present during sporulation of the oocysts.

Proteins C, D, E, F, G, H, I, J, K, L, and M are all produced at least during the development of the oocyst through the sporozoite stage. Protein D has been found to be present in sporozoites, merozoites and schizonts and is believed to be actively synthesized in each of these stages. Protein H first appears approximately 8 hours into sporulation of the oocyst and persists into the sporozoite stage.

Protein G contains at least 5, preferably at least 21 and more preferably at least 45 glutamine residues. Such a preponderence of glutamine residues is a feature believed to be unique to Eimeria proteins. It is believed that these glutamine residues are in a direct sequence and that at least some additional amino acid residues may be appended to either the amino or carboxylic terminus of the polyglutamine sequence.

Moreover, it is believed that each of these proteins contain one or more specific portions which may serve as antigenic determinants capable of binding to at least one antibody present in sera of Eimeria infected chickens. These specific antigenic portions, either singly or in various combinations are, therefore, capable of serving as the basis for an in vitro diagnostic assay.

DNA sequences encoding these proteins are contained in the EMBL phage clones identified herein. The DNA sequences encoding for the proposed specific antigenic portions of these proteins are also contained in the EMBL phage clones. Table 1 indicates the relationship between the proteins, the EMBL phage clones containing the Eimeria DNA encoding the proteins and the insert clones encoding the proposed specific antigenic portions of the proteins.

Table 1

| Polypeptide | Molecular Weight (kd) in Sporozoite | Lambda EMBL Phage Clone | Plasmid Insert Clone | Plasmid Insert Size (bp) | Type of Insert (cDNA or genomic) |
|---|---|---|---|---|---|
| A | 120 | ET::Rbl-8 | Rbl-8 | 1900 | cDNA |
| B | 100 | ET::Rbl-17 | Rbl-17 | 1700 | cDNA |
| C | 200 | ET::CH2-13 | CH2-13 | 2400 | cDNA |
| D | 80 | ET::CH2-15 | CH2-15 | 725 | cDNA |
| | | | GRS-84 | 310 | Genomic |
| | | | CH2-6 | 870 | cDNA |
| | | | GRS-53 | 1600 | Genomic |
| E | 80 | ET::Rbl-145 | Rbl-145 | 640 | cDNA |
| F | 45 | ET::GRS-74 | GRS-74 | 600 | Genomic |
| | | | GRS-77 | 900 | Genomic |
| G | 30 | ET::CHAD-19 | CHAD-19 | 270 | cDNA |
| | | | GRS-15 | 275 | Genomic |
| | | | GRS-72 | 450 | Genomic |
| | | | GRS-45 | 3000 | Genomic |
| H | 25 | ET::CAL-2a | Cal-2a | 280 | cDNA |
| | | | Cal-2cn | 280 | cDNA |
| I | 14 | ET::CAL-3gn | Cal-3gn | -- | cDNA |
| | | | Cal-3in | 970 | cDNA |
| J | 10 | ET::CAL-4cn | Cal-4cn | 2000 | cDNA |
| | | | Rbl-124 | 2000 | cDNA |
| K | | ET::Rbl-19 | Rbl-19 | 750 | cDNA |
| L | | ET::Rbl-24 | Rbl-24 | 450 | cDNA |
| M | | ET::CH2-3 | CH2-3 | 270 | cDNA |

Restriction maps for certain of the EMBL clones listed in Table 1 are depicted in the Figures. In the Figures, the following restriction sites are indicated by the abbreviations designated below for the corresponding restriction endonucleases:

| Endonuclease | Abbreviation |
|---|---|
| EcoRI | R |
| SalI | S |
| HindIII | H |
| BamHI | B |
| KpnI | K |

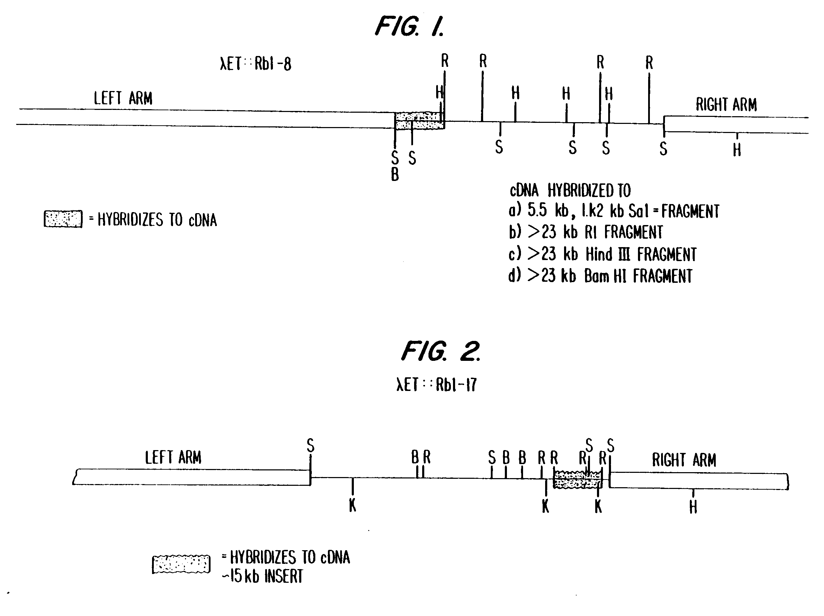

Fig. 1 is a restriction enzyme map of a lambda ET phage containing Eimeria DNA that encodes Protein A. The region of the ET phage corresponding to cDNA clone Rbl-8 is depicted. Subcloning the Rbl-8 cDNA insert into an appropriate expression vector (see Section IV) will result in synthesis of a portion of Protein A. DNA that directs synthesis of the entire Protein A polypeptide is contained within the Eimeria DNA portion of the phage and can be identified as described in Section VI.

Fig. 2 is a restriction enzyme map of a lambda ET phage containing Eimeria DNA that encodes Protein B. The region of the ET phage corresponding to cDNA clone Rbl-17 is depicted. Subcloning the Rbl-17 cDNA insert into an appropriate expression vector (see Section IV) will result in synthesis of a portion of Protein B. DNA that directs synthesis of the entire Protein B polypeptide is contained within the Eimeria DNA portion of the phage and can be identified as described in Section VI. Lambda phage ET::Rbl-17 has also been deposited with the American Type Culture Collection (ATCC) under Accession No. 40273.

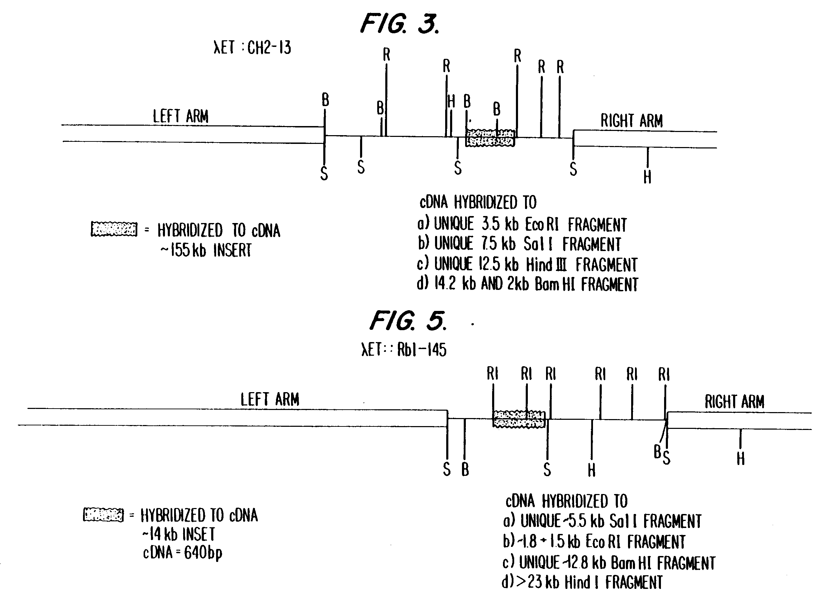

Fig. 3 is a restriction enzyme map of a lambda ET phage containing Eimeria DNA that encodes Protein C. The region of the ET phage corresponding to cDNA clone CH2-13 is depicted. Subcloning the CH2-13 cDNA insert into an appropriate expression vector (see Section IV) will result in synthesis of a portion of Protein C. DNA that directs synthesis of the entire Protein C polypeptide is contained within the Eimeria DNA portion of the phage and can be identified as described in Section VI.

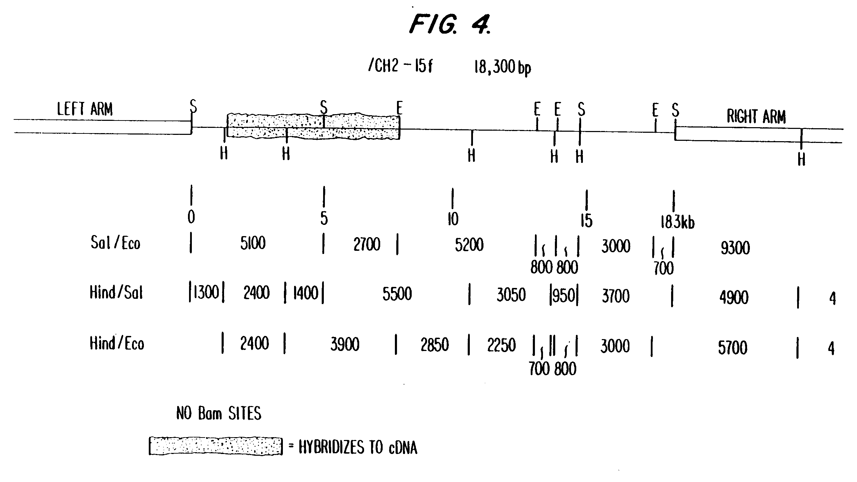

Fig. 4 is a restriction enzyme map of a lambda ET phage containing Eimeria DNA that encodes Protein D. The region of the ET phage corresponding to cDNA clone Ch2-15 is depicted. Subcloning the Ch2-15 cDNA insert into an appropriate expression vector (see Section IV) will result in synthesis of a portion of Protein D. DNA that directs synthesis of the entire Protein D polypeptide is contained within the Eimeria DNA portion of the phage and can be identified as described in Section VI.

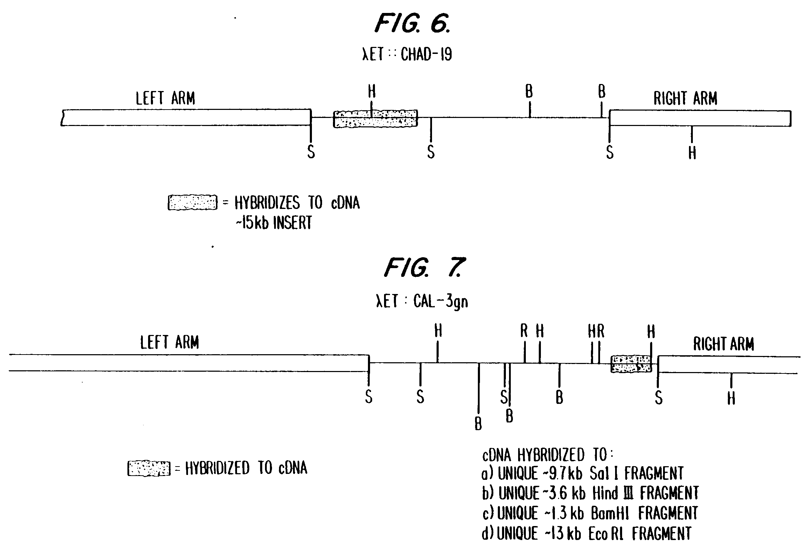

Fig. 5 is a restriction enzyme map of a lambda ET phage containing Eimeria DNA that encodes Protein E. The region of the ET phage corresponding to cDNA clone Rbl-145 is depicted. Subcloning the Rbl-145 cDNA insert into an appropriate expression vector (see Section IV) will result in synthesis of a portion of Protein E. DNA that directs synthesis of the entire Protein E polypeptide is contained within the Eimeria DNA portion of the phage and can be identified as described in Section VI.

Fig. 6 is a restriction enzyme map of a lambda ET phage containing Eimeria DNA that encodes Protein G. The region of the ET phage corresponding to cDNA clone CHAD-19 is depicted. Subcloning the CHAD-19 cDNA insert into an appropriate expression vector (see Section IV) will result in synthesis of a portion of Protein G. DNA that directs synthesis of the entire Protein G polypeptide is contained within the Eimeria DNA portion of the phage and can be identified as described in Section VII.

As noted previously, Protein G contains the "repeat" glutamine sequences. In addition to CHAD-19, other DNA sequences have been found which encode repeat glutamine sequences. These include GRS-15, GRS-72, GRS-45, GRS-74, GRS-77 and CH2-3. Inserts GRS-77 and CH2-3 have been described previously. The remainder of these DNA sequences and any other Eimera tenella DNA sequences may be isolated according to the methods set forth in Example 7.

Fig. 7 is a restriction enzyme map of a lambda ET phage containing Eimeria DNA that encodes Protein I. The region of the ET phage corresponding to cDNA clone Cal-3gn is depicted. Subcloning the Cal-3gn cDNA insert into an appropriate expression vector (see Section IV) will result in synthesis of a portion of Protein I. DNA that directs synthesis of the entire Protein I polypeptide is contained within the Eimeria DNA portion of the phage and can be identified as described in Section VI.

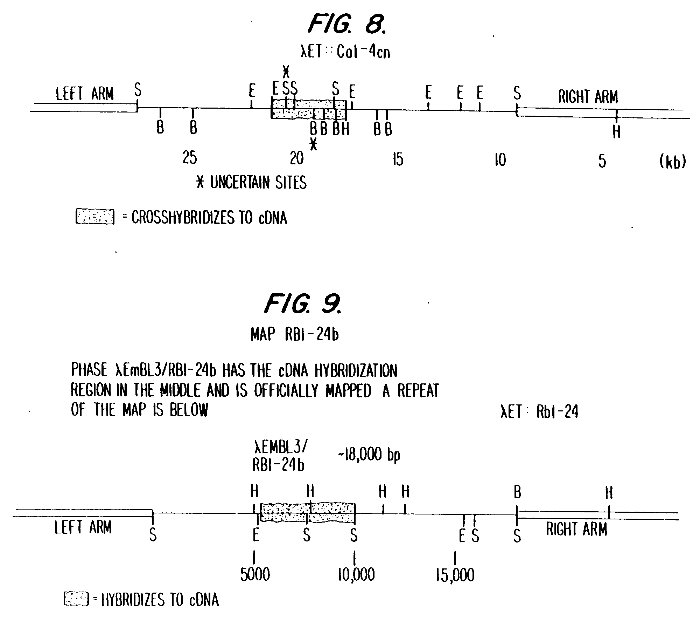

Fig. 8 is a restriction enzyme map of a lambda ET phage containing Eimeria DNA that encodes Protein J. The region of the ET phage corresponding to cDNA clone Cal-4cn is depicted. Subcloning the Cal-4cn cDNA insert into an appropriate expression vector (see Section IV) will result in synthesis of a portion of Protein J. DNA that directs synthesis of the entire Protein J polypeptide is contained within the Eimeria DNA portion of the phage and can be identified as described in Section VI.

Fig. 9 is a restriction enzyme map of a lambda ET phage containing Eimeria DNA that encodes Protein L. The region of the ET phage corresponding to cDNA clone Rbl-24 is depicted. Subcloning the Rbl-24 cDNA insert into an appropriate expression vector (see Section IV) will result in synthesis of a portion of Protein L. DNA that directs synthesis of the entire Protein L polypeptide is contained within the Eimeria DNA portion of the phage and can be identified as described in Section VI.

A lambda ET phage containing Eimeria DNA that encodes Protein H has been deposited with the ATCC under Accession No. 40270. The cDNA clone is called Cal-2a.

A lambda ET phage containing Eimeria DNA that encodes Protein K has been deposited with the ATCC under Accession No. 40272. The cDNA clone is called Rbl-19.

A lambda ET phage containing Eimeria DNA that encodes Protein M has been deposited with the ATCC under Accession No. 40269. The cDNA clone is called CH2-3.

The proteins described herein may be manufactured by the recombinant DNA methods set forth more fully below, using DNA sequences contained on the associated EMBL phage clones as set forth in Table 1. Additionally, the DNA sequences corresponding to the proposed antigenic portions of the proteins contained in the EMBL phage clones may be utilized in recombinant DNA methods to create the amino acid sequences which, it is believed, form certain of the antigenic determinants of the proteins.

Various methods may be used to express the DNA encoding the proteins or the proposed antigenic determinants. In particular, it is contemplated that the DNA contained on the EMBL phage clones may be expressed in mammalian systems. These systems include, for example, viral vector delivery systems as set forth in Eukaryotic Viral Vectors edited by Y. Gluzman, Cold Spring Harbor Laboratory Cold Spring Harbor, New York (1982), specifically incorporated herein by reference.

In an alternate preferred embodiment, it is contemplated that the DNA of interest will be excised from the EMBL phage clone and inserted, in a suitable form, into a microbial expression system. In this embodiment, the antigenic polypeptides are produced by a method comprising:

(a) preparation of a DNA sequence capable of directing a host microorganism to produce a polypeptide possessing antigenic properties analogous to those possessed by a polypeptide produced by Eimeria organisms;

(b) cloning the DNA sequence into a vector capable of being transferred into and replicating in a host microorganism, such vector containing operational elements for the DNA sequence;

(c) transferring the vector containing the DNA sequence and operational elements into a host microorganism capable of expressing the antigenic polypeptides;

(d) culturing the host microorganism under conditions appropriate for amplification of the vector and expression of the polypeptide; and

(e) in either order:

(i) harvesting the polypeptide; and

(ii) causing the polypeptide to assume a structure whereby it possesses antigenic properties analogous to properties possessed by polypeptides produced by Eimeria organisms.

In this method, the DNA is prepared either by removal of any introns present in the lambda ET phage sequence encoding the polypeptide or by identifying a cDNA clone capable of hybridizing to the lambda ET phage sequence of interest. In the first method of preparing the DNA, the 5' and 3' gene limits are identified and it is determined whether any intervening sequences, i.e., introns, are present. These intervening sequences are readily identifiable to one of ordinary skill in the art in light of the teaching and phage maps contained herein and the available literature such as Berk, A.J. and Sharp, P.A. in Cell 12:721(1977), specifically incorporated herein by reference. For example, the presence of an intervening sequence may be determined by ascertaining the nucleotide sequence of the DNA encoding the protein and examining that sequence to identify nucleotide sequences known to occur in introns. Any identified introns could then be excised by bridging the intron with an oligonucleotide and cleaving the intron sequence with an appropriate nuclease such as SI nuclease. Examples of the SI nuclease procedure are set forth in Berk, A.J. and Sharp, P.A. in Cell 12:721(1977), specifically incorporated herein by reference.

In an alternate method of preparing the DNA for expression in a microbial system, the portion of the lambda ET phage of interest is identified in light of the phage maps contained herein. This portion of the lambda phage, or a polynucleotide subset thereof is used to probe a cDNA library created from Eimeria mRNA obtained from an appropriate stage of the Eimeria life cycle. Preferably, the mRNA is obtained from sporozites. A preferred method for the creation of the Eimeria cDNA library is set forth in the Examples below. The hybridizing cDNA clone thus identified is suitable for use in the microbial expressior system.

The DNA prepared in accordance with the above methods is inserted into an expression vector suitable for use in the intended expression system. Embodiments of the present invention are envisioned as employing other known or currently undiscovered vectors which would contain one or more of the DNA sequences encoding antigenic polypeptides described herein. In particular, it is preferred that these vectors have some or all of the following characteristics: (1) possess a minimal number of host-organism sequences; (2) be stable in the desired host; (3) be capable of being present in a high copy number in the desired host; (4) possess a regulatable promoter; (5) have at least one DNA sequence coding a selectable trait present on a portion of the plasmid separate from that where the DNA sequence encoding for the antigenic polypeptide will be inserted; and (6) be integrated into the vector.

The following, noninclusive, list of cloning vectors and operational elements for E. coli and other organisms is believed to set forth vectors which can easily be altered to meet the above-criteria and are therefore preferred for use in the present invention. Such alterations are easily performed by those of ordinary skill in the art in light of the available literature and the teachings herein.

TABLE 2

| Host | Regulated Promoters | Inducer | Transcription Terminator | mRNA Stabilization | Transcriptional Start Site & Leader Peptide | Marker | RS Binding Site |
|---|---|---|---|---|---|---|---|
| E. coli | Lac[1], Tac[2] | IPTG, | rrnB[6] | ompA[8] | bla[11] | ampicillin[14] | |
| | Lambda pL [3,4] | increased temperature, | rrnC[7] | lambda int[9] | ompA[12] | tetracycline[14,15] | |
| | Trp[5] | IAA addition, or | | trp[10] | phoS[13] | chloramphenicol[16] | |
| | | tryptophan depletion | | | | | |
| Bacillus | *alpha amylase[17] | | E. coli rrn | | B.amy neutral protease[21] | Kan[r 24] | B.amy neutra protease |
| | *subtilisin18 | | BT.T20 | | | Cam[r 25§] | |
| | *p-4319 | | | | B.amy alpha-amylase22 | | |
| | spac-126 | IPTG | | | | | B.amy alpha-amylase[22] |
| | | | | | B.subt. subtilisin23 | | |
| | Trp27 (E. coli) | IAA addition, or | | | phospholipase C28 | sulfonamide30 | Trp (E. coli) |
| | Lac (E. coli) | tryptophan depletion | | | exotoxin A29 | streptomycin30 | |
| Pseudo-monas | Tac (E. coli) | IPTG | | | | | |
| Yeast | Gal 131, 1032 | Glucose depletion and galactose | Cyc 1 | | Invertase36 | Ura 337 | |
| | | | Una | | Acid phosphatase36 | Leu 238 | |
| | Adh 133, 1134 | Glucose depletion | Alpha factor | | | | |
| | Pho 535 | Phosphate depletion | Suc 2 | | Alpha Factor | His 3 | |
| | | | | | | Tap 139 | |

*non-regulated

1. Backman, K., Ptashne, M. and Gilbert, W. Proc. Natl. Acad. Sci. USA 73, 4174-4178 (1976).

2. de Boer, H.A., Comstock, L.J., and Vasser, M. Proc. Natl. Acad. Sci. USA 80, 21-25 (1983).

3. Shimatake, H. and Rosenberg, M. Nature 292, 128-132 (1981).

4. Derom, C., Gheysen, D. and Fiers, W. Gene 17, 45-54 (1982).

5. Hallewell, R.A. and Emtage, S. Gene 9, 27-47 (1980).

6. Brosius, J., Dull, T.J., Sleeter, D.D. and Noller, H.F. J. Mol. Biol. 148, 107-127 (1981).

7. Normanly, J., Ogden, R.C., Horvath, S.J. and Abelson, J. Nature 321, 213-219 (1986).

8. Belasco, J.G., Nilsson, G., von Gabain, A. and Cohen, S.N. Cell 46, 245-251 (1986).

9. Schmeissner, U., McKenney, K., Rosenberg M. and Court, D. J. Mol. Biol. 176, 39-53 (1984).

10. Mott, J.E., Galloway, J.L. and Platt, T. EMBO J. 4, 1887-1891 (1985).

11. Koshland, D. and Botstein, D. Cell 20, 749-760 (1980).

12. Movva, N.R., Nakamura, K. and Inouye, M. J. Mol. Biol. 143, 317-328 (1980).

13. Surin, B.P., Jans, D.A., Fimmel, A.L., Shaw, D.C., Cox, G.B. and Rosenberg, H. J. Bacteriol. 157, 772-778 (1984).

14. Sutcliffe, J.G. Proc. Natl. Acad. Sci. USA 75, 3737-3741 (1978).

15. Peden, K.W.C. Gene 22, 277-280 (1983).

16. Alton, N.K. and Vapnek, D. Nature 282, 864-869 (1979).

17. Yang, M., Galizzi, A., and Henner, D. Nuc. Acids Res. 11(2), 237-248 (1983).

18. Wong, S.-L., Price, C.W., Goldfarb, D.S., and Doi, R.H. Proc. Natl. Acad. Sci. USA 81, 1184-1188 (1984).

19. Wang, P.-Z., and Doi, R.H. J. Biol. Chem. 259, 8619-8625, (1984).

20. Lin, C.-K., Quinn, L.A. Rodriquez, R.L. J. Cell Biochem. Suppl. (9B), p.198 (1985).

21. Vasantha, N., Thompson, L.D., Rhodes, C., Banner, C., Nagle, J., and Filpula, D. J. Bact. 159(3), 811-819 (1984).

22. Palva, I., Sarvas, M., Lehtovaara, P., Sibazkov, M., and Kaariainen, L. Proc. Natl. Acad. Sci. USA 79, 5582-5586 (1982).

23. Wong. S.-L., Pricee, C.W., Goldfarb, D.S., and Doi, R.H. Proc. Natl. Acad. Sci. USA 81, 1184-1188 (1984).

24. Sullivan, M.A., Yasbin, R.E., and Young, F.E. Gene 29, 21-46 (1984).

25. Vasantha, N., Thompson, L.D., Rhodes, C., Banner, C. Nagle, J., and Filpula, D. J. Bact. 159(3), 811-819 (1984).

26. Yansura, D.G. and Henner, D.J. PNAS 81, 439-443 (1984).

27. Gray, G.L., McKeown, K.A., Jones, A.J.S., Seeburg, P.H. and Heyneker, H.L. Biotechnology, 161-165 (1984).

28. Lory, S., and Tai, P.C. Gene 22, 95-101 (1983).

29. Liu, P.V. J. Infect. Dis. 130 (suppl), 594-599 (1974).

30. Wood, D.G., Hollinger, M.F., and Tindol, M.B. J. Bact. 145, 1448-1451 (1981).

31. St. John, T.P. and Davis, R.W. J. Mol. Biol. 152, 285-315 (1981).

32. Hopper, J.E., and Rowe, L.B. J. Biol. Chem. 253, 7566-7569 (1978).

33. Denis, C.L., Ferguson, J. and Young, E.T. J. Biol. Chem. 258, 1165-1171 (1983).

34. Lutsdorf, L. and Megnet, R. Archs. Biochem. Biophys. 126, 933-944 (1968).

35. Meyhack, B., Bajwa, N., Rudolph, H. and Hinnen, A. EMBO. J. 6, 675-680 (1982).

36. Watson, M.E.E. Nucleic Acid Research 12, 5145-5164 (1984).

37. Gerband, C. and Guerineau, M. Curr. Genet. 1, 219-228 (1980).

38. Hinnen, A., Hicks, J.B. and Fink, G.R. Proc. Natl. Acad. Sci. USA 75, 1929-1933 (1978).

39. Jabbar, M.A., Sivasubramanian, N. and Nayak, D.P. Proc. Natl. Acad. Sci. USA 82, 2019-2023 (1985).

It is to be understood that additional cloning vectors may now exist or will be discovered which have the above-identified properties and are therefore suitable for use in the present invention. These vectors are also contemplated as being within the scope of the disclosed series of cloning vectors into which the cDNA sequences may be introduced, along with any necessary operational elements, and which altered vector is then included within the scope of the present invention and would be capable of being used in the recombinant-DNA method set forth more fully below.

A. Characteristics of Preferred Vectors

Certain embodiments of the present invention are envisioned which employ known or currently undiscovered vectors which would contain one or more of the cDNA sequences described herein. In particular, it is preferred that these vectors have some or all of the following characteristics: (1) possess a minimal number of host-organism sequences; (2) be stably maintained and propagated in the desired host; (3) be capable of being present in a high copy number in the desired host; (4) possess a regulatable promoter positioned so as to promote transcription of the gene of interest; (5) have at least one DNA sequence coding for a selectable trait present on a portion of the plasmid separate from that where the portable DNA sequence will be inserted; and (6) a DNA sequence capable of terminating transcription.

In various preferred embodiments, these cloning vectors containing and capable of expressing the cDNA sequences of the present invention contain various operational elements. These "operational elements," as discussed herein, include at least one promoter, at least one Shine-Dalgarno sequence and initiator codon, and at least one terminator codon. Preferably, these "operational elements" also include at least one operator, at least one leader sequence for proteins to be exported from intracellular space, at least one gene for a regulator protein, and any other DNA sequences necessary or preferred for appropriate transcription and subsequent translation of the vector DNA.

Certain of these operational elements may be present in each of the preferred vectors of the present invention. It is contemplated any additional operational elements which may be required may be added to these vectors using methods known to those of ordinary skill in the art, particularly in light of the teachings herein.

In practice, it is possible to construct each of these vectors in a way that allows them to be easily isolated, assembled, and interchanged. This facilitates assembly of numerous functional genes from combinations of these elements and the coding region of the cDNA sequences. Further, many of these elements will be applicable in more than one host. It is additionally contemplated that the vectors, in certain preferred embodiments, will contain DNA sequences capable of functioning as regulators ("operators"), and other DNA sequences capable of coding for regulator proteins.

## 1. Regulators

These regulators, in one embodiment, will serve to prevent expression of the cDNA sequences in the presence of certain environmental conditions and, in the presence of other environmental conditions, will allow transcription and subsequent expression of the protein coded for by the cDNA sequence. In particular, it is preferred that regulatory segments be inserted into the vector such that expression of the cDNA sequence will not occur, or will occur to a greatly reduced extent, in the absence of, for example, isopropylthio- D-galactoside. In this situation, the transformed microorganisms containing the cDNA sequence may be grown to a desired density prior to initiation of the expression of Proteins A through N. In this embodiment, expression of the desired trypsin inhibitor is induced by addition of a substance to the microbial environment capable of causing expression of the DNA sequence after the desired density has been achieved.

## 2. Promoters

The expression vectors must contain promoters which can be used by the host organism for expression of its own proteins. While the lactose promoter system is used commonly, other microbial promoters have been isolated and characterized, enabling one skilled in the art to use them for expression of the Protein A through N.

## 3. Transcription Terminator

The transcription terminators contemplated herein serve to stabilize the vector. In particular, those sequences as described by Rosenberg, M. and Court, D., in Ann. Rev. Genet. 13:319-353 (1979), specifically incorporated herein by reference, are contemplated for use in the present invention.

## 4. Non-Translated Sequence

It is noted that, in the preferred embodiment, it may also be desirable to reconstruct the 3' or 5' end of the coding region to allow incorporation of 3' or 5' non-translated sequences into the gene transcript. Included among these non-translated sequences are those which stabilize the mRNA as they are identified by Schmeissner, U., McKenney, K., Rosenberg, M. and Court, D. in J. Mol. Biol. 176:39-53 (1984), specifically incorporated herein by reference.

### 5. Ribosome Binding Sites

The microbial expression of foreign proteins requires certain operational elements which include, but are not limited to, ribosome binding sites. That particular element is a sequence which a ribosome recognizes and binds to in the initiation of protein synthesis as set forth in Gold, L., et al., Ann. Rev. Microbiol. 35:557-580 (1983); Marquis, D.M., et al., Gene 42:175-183 (1986), specifically incorporated herein by reference.

The operational elements as discussed herein can be routinely selected by those of ordinary skill in the art in light of prior literature and the teachings contained herein. General examples of these operational elements are set forth in B. Lewin, Genes, Wiley & Sons, New York (1983), which is specifically incorporated herein by reference. Various examples of suitable operational elements may be found on the vectors discussed above and may be elucidated through review of the publications discussing the basic characteristics of the aforementioned vectors.

### 6. Leader Sequence and Translational Coupler

Additionally, it is preferred that DNA coding for an appropriate secretory leader (signal) sequence be present at the 5' end of the portable DNA sequence. The DNA for the leader sequence must be in a position which allows the production of a fusion protein in which the leader sequence is immediately adjacent to and covalently joined to the inhibitor, i.e., there must be no transcription or translation termination signals between the two DNA coding sequences. The presence of the leader sequence is desired in part for one or more of the following reasons. First, the presence of the leader sequence may facilitate host processing of Proteins A through N. In particular, the leader sequence may direct cleavage of the initial translation product by a leader peptidase to remove the leader sequence and leave a polypeptide with the amino acid sequence which has potential protein activity. Second, the presence of the leader sequence may facilitate purification of the Protein A through N, through directing the protein out of the cell cytoplasm. In some species of host microorganisms, the presence of the appropriate leader sequence will allow transport of the completed protein into the periplasmic space, as in the case of some E. coli. In the case of certain E. coli, saccharomyces and strains of Bacillus and Pseudomonas, the appropriate leader sequence will allow transport of the protein through the cell membrane and into the extracellular medium. In this situation, the protein may be purified from extracellular protein. Thirdly, in the case of some of the proteins prepared by the present invention, the presence of the leader sequence may be necessary to locate the completed protein in an environment where it may fold to assume its active structure, which structure possesses the appropriate protein activity.

### 7. Translation Terminator

The translation terminators contemplated herein serve to stop the translation of mRNA. They may be either natural, Kohli, J., Mol. Gen. Genet. 182:430-439 (1981), or synthesized, Pettersson, R.F., Gene 24:15-27 (1983), both of which references are specifically incorporated herein by reference.

### 8. Selectable Marker

Additionally, it is preferred that the cloning vector contain a selectable marker, such as a drug resistance marker or other marker which causes expression of a selectable trait by the host microorganism. In one embodiment of the present invention, the gene for ampicillin resistance is included in the vector while in other plasmids, the gene for tetracycline resistance or the gene for chloramphenicol resistance is included.

Such a drug resistance or other selectable marker is intended in part to facilitate in the selection of transformants. Additionally, the presence of such a selectable marker on the cloning vector may be of use in keeping contaminating microorganisms from multiplying in the culture medium. In this embodiment, such a pure culture of the transformed host microorganisms would be obtained by culturing the microorganisms under conditions which require the induced phenotype for survival.

### B. Vector Assembly

Upon synthesis and/or isolation of all necessary and desired component parts of the above-discussed cloning vectors, the vectors are assembled by methods generally known to those of ordinary skill in the art.

Assembly of such vectors is believed to be within the duties and tasks performed by those with ordinary skill in the art and, as such, is capable of being performed without undue experimentation. For example, similar DNA sequences have been ligated into appropriate cloning vectors, as set forth in Maniatis, T., et al. Molecular Cloning: A Laboratory Manual, Cold Spring Harbor Laboratory, N.Y. (1982), which is specifically incorporated herein by reference.

## C. Host Organisms

The vectors and methods disclosed here are suitable for use in host cells over a wide range of prokaryotic and eukaryotic organisms. Prokaryotes are preferred for the cloning of DNA sequences and for the expression of the gene. The E. coli strains JM105, JM107 and JM109 (available from Pharmacia), as well as Bacilli, and Pseudomonas species, may be used for expression of the gene. In addition to prokaryotes, eukaryotic microbes such as Saccharomyces cerevisiae may be used for expression of the gene.

In general, plasmid vectors containing operational elements which are derived from species compatible with the host cells are used. For example. E. coli is typically transformed using pBR322, a plasmid derived from E. coli. Table 2 indicates the list of host organisms and the compatible vectors.

### 1. Pseudomonas Vectors

In addition to the vectors listed in Table 2, several vector plasmids which autonomously replicate in a broad range of Gram Negative bacteria are preferred for use as cloning vehicles in hosts of the genera Pseudomonas. These are described by Tait, R.C., Close, T.J., Lundquist, R.C., Hagiya, M., Rodriguez, R.L., and Kado, C.I. in Biotechnology, May, 1983, pp. 269-275; Panopoulos, N.J. in Genetic Engineering in the Plant Sciences, Praeger Publishers, New York, New York, pp. 163-185, (1981); and Sakaguchi, K. in Current Topic in Microbiology and Immunology 96:31-45, (1982), each of which is specifically incorporated herein by reference.

One particularly preferred construction would employ the plasmid RSF1010 and derivatives thereof as described by Bagdasarian, M., Bagdasarian, M.M., Coleman, S., and Timmis, K.N. in Plasmids of Medical Environmental and Commercial Importance, Timmis, K.N. and Puhler, A. eds., Elsevier/North Holland Biomedical Press, (1979), specifically incorporated herein by reference. The advantages of RSF1010 are that it is relatively small, high copy number plasmid which is readily transformed into and stably maintained in both E. coli and Pseudomonas species. In this system, it would be preferred to use the Tac expression system as described for Escherichia, since it appears that the E. coli trp promoter is readily recognized by Pseudomonas RNA polymerase as set forth by Sakaguchi, K. in Current Topics in Microbiology and Immunology 96:31-45 (1982) and Gray, G.L., McKeown, K.A., Jones, A.J.S., Seeburg, P.H., and Heyneker, H.L. in Biotechnology Feb. 1984, pp. 161-165, both of which are specifically incorporated herein by reference. Transcriptional activity may be further maximized by requiring the exchange of the promoter with, e.g., an E. coli or P. aeruginosa trp promoter. Additionally, the laclq gene of a laclq strain of E. coli would also be included in the plasmid to effect regulation.

Translation may be coupled to translation initiation for any of the E. coli proteins as described in the Examples, as well as to initiation sites for any of the highly expressed proteins of the host to cause intracellular expression of the inhibitor.

In those cases where restriction minus strains of a host Pseudomonas species are not available, transformation efficiency with plasmid constructs isolated from E. coli are poor. Therefore, passage of the Pseudomonas cloning vector through an r- m+ strain of another species prior to transformation of the desired host, as set forth in Bagdasarian, M., et al., Plasmids of Medical, Environmental and Commercial Importance, pp. 411-422, Timmis and Puhler eds., Elsevier,/North Holland Biomedical Press (1979), specifically incorporated herein by reference, is desired.

### 2. Bacillus Vectors

Furthermore, a preferred expression system in hosts of the genera Bacillus involves using plasmid pUB110 as the cloning vehicle. As in other host vector systems, it is possible in Bacillus to express the trypsin inhibitors of the present invention as either an intracellular or a secreted protein. The present embodiments include both systems. Shuttle vectors that replicate in both Bacillus and E. coli are available for constructing and testing various genes as described by Dubnau, D., Gryczan, T., Contente, S., and Shivakumar, A.G. in Genetic Engineering, Vol. 2, Setlow and Hollander eds., Plenum Press, New York, New York, pp. 115-131, (1980), specifically incorporated herein by reference. For the expression and secretion of trypsin inhibitors from B. subtilis, the signal sequence of alpha-amylase is preferably coupled to the coding region for the inhibitor. (For synthesis of intracellular inhibitor, the portable DNA sequence will be translationally coupled to the ribosome binding site of the alpha-amylase leader sequence).

Transcription of either of these constructs is preferably directed by the alpha-amylase promoter or a derivative thereof. This derivative contains the RNA polymerase recognition sequence of the native alpha-amylase promoter but incorporates the lac operator region as well. Similar hybrid promoters constructed from the penicillinase gene promoter and the lac operator have been shown to function in Bacillus hosts in a regulatable fashion as set forth by Yansura, D.G. and Henner in Genetics and Biotechnology of Bacilli, Ganesan, A.T. and Hoch, J.A., eds., Academic Press, pp. 249-263, (1984), specifically incorporated by reference. The lacI gene of a lacIq strain of E. coli would also be included in the placement to effect regulation.

### 3. Clostridium Vectors

One preferred construction for expression in Clostridium is in plasmid pJU12 described by Squires, C. H. et al in J. Bacteriol. 159:465-471 (1984), specifically incorporated herein by reference, transformed into C. perfringens by the method of Heefner, D. L. et al. as described in J. Bacteriol. 159:460-464 (1984), specifically incorporated herein by reference. Transcription is directed by the promoter of the tetracycline resistance gene. Translation is coupled to the Shine-Dalgarno sequences of this same tet$^r$ gene in a manner strictly analogous to the procedures outlined above for vectors suitable for use in other hosts.

### 4. Yeast Vectors

Maintenance of foreign DNA introduced into yeast can be effected in several ways (Botstein, D., and Davis, R. W., in The Molecular Biology of the Yeast Saccharomyces, Cold Spring Harbor Laboratory, Strathern, Jones and Broach, eds., pp. 607-636 (1982). One preferred expression system for use with host organisms of the genus Saccharomyces harbors the trypsin inhibitor gene on the 2 micron plasmid. The advantages of the 2 micron circle include relatively high copy number and stability when introduced into cir° strains. These vectors preferably incorporate the replication origin and at least one antibiotic resistance marker from pBR322 to allow replication and selection in E. coli. In addition, the plasmid will preferably have 2 micron sequences and the yeast LEU2 gene to serve the same purposes in LEU2 defective mutants of yeast.

### EXAMPLES

#### I. CONSTRUCTION OF THE LAMBDA GT11 EXPRESSION LIBRARIES

A. Construction of The "Small Fragment" E. Tenella
Genomic DNA Expression Library

The rationale for construction of a "small fragment" genomic expression library was to obtain a representative clone for every antigenically active epitope that could be expressed by E. tenella. By using small genomic fragments, the probability of a peptide determinant folding in a way that approximates its in vivo state may be increased. A large number of clones are required to adequately represent the E. tenella genome. Using 3 X 10$^8$ bp as the estimate of the genome size for E. tenella, it was calculated that for a 99% probability of having every 300 bp fragment in both orientations and in all three reading frames, 3 x 10$^7$ individual recombinants were required.

Because a large number of recombinants are required for a representative genomic expression library, it was decided to use the lambda gt11 system as described by Young, R. and Davis, R. in PNAS 80:1194 (1983). Through use of the lambda gt11 system, large numbers of recombinants could be obtained due to the highly efficient in vitro packaging systems that have been developed for lambda. In lambda gt11, cloned DNA fragments coding for peptides are expressed as fusion proteins when they are inserted at the unique EcoRI site near the carboxy terminus of the beta-galactosidase gene in the phage. Insertion of foreign DNA in the beta-galactosidase gene inactivates the gene, thus allowing identification of recombinant phages on indicator plates.

The "small fragment" E. tenella genomic expression library was obtained in four consecutive steps. First, lambda gt11 DNA was purified and prepared for efficient cloning of insert DNA fragments. Second, genomic DNA was obtained from E. tenella sporozoites. Third, "small fragments" of the E. tenella genome were prepared by cleavage with RsaI restriction endonuclease, modification with EcoRI methylase and subsequent frame adaptation with different length oligonucleotides. Fourth, the prepared "small fragments" were ligated into the lambda gt11 vector DNA and packaged in vitro. Finally, the in vitro packaged recombinant phage were amplified.

1. Purification and Preparation of Lambda gt11 DNA

A lysate of lambda gt11 was obtained from Dr. D. Hirsh (MCD Biology Dept., University of Colorado). The phage was plated on the host E. coli strain KM392 which contains a lac deletion. Plate lysates of this phage were prepared using the methods described by Davis, R., et al. in 1981. The gt11 phage were concentrated from the lysate by centrifugation at 20,000 rpm, using a Beckman JA-20 rotor, for 3 hour. The concentrated phage were purified by sedimentation through Cesium Chloride (CsCl) gradients as described in Section III (part VB page 58), infra. The DNA was extracted from the phage particles by exhaustive dialysis against 50% formamide (MCB) and subsequent dialysis against DNA storage buffer 10mM Tris-HCl(pH7.5);1mM EDTA (TE buffer)

Since there is no genetic selection for recombinant phage with the lambda gt11 system, a biochemical selection was used. In order to "protect" the cohesive ends of the phage during this selection, the following reactions were done. First, the purified DNA was heated to 70°C, cooled to 42°C, and the cohesive ends of the phage were allowed to anneal for 3 hour. The mixture was then cooled to 14°C and a 1/10 volume of 10X ligase buffer was added, followed by T4 DNA ligase (P.L. Biochemicals). The ligation reaction was conducted for 14 hour at 14°C.

After the gt11 arms were sealed, the DNA was treated with an excess of EcoRI. After digestion, the EcoRI was inactivated by heating the reaction mix at 70°C for 10 minutes, then calf intestinal alkaline phosphatase (Boehringer-Mannheim) was added and the reaction mix incubated at 37°C for 15 minutes. The quantity of alkaline phosphatase was empirically determined to give a low background level after ligation and packaging. To ensure that the EcoRI ends of the vector were intact, recovery of beta-galactosidase activity in lambda gt11 was monitored after treating the EcoRI ends with polynucleotide kinase (P.L. Biochemicals) and T4 DNA ligase (P.L. Biochemicals). The alkaline phosphatase was removed from the vector DNA preparation by three phenol extractions followed by three ether extractions. The extracted vector DNA was then made 0.3 M with sodium acetate, and 2.5 volumes of ethanol were added to precipitate the DNA. The precipitated DNA was resuspended in a small volume of Tris-EDTA (TE) buffer (10 mM Tris-HCl, pH 7.5; 1 mM EDTA). A typical phosphatase treatment of gt11 phage DNA decreased the infectivity of the phage by at least two logs. The dephosphorylated molecules cannot religate to themselves, and the only way to obtain biological activity is to insert a DNA molecule between the two phage arms.

2. Preparation of Genomic DNA From Eimeria Tenella Sporozoites

DNA was obtained from E. tenella using protocols such as those described by Maniatis, T., et al. in Cell 15:687 (1978) and Blin, N. and Stafford, D.W. in Nucleic Acids Res. 3:2303 (1976). Approximately $5 \times 10^9$ frozen E. tenella sporozoites were thawed on ice and transferred to a plastic tube. The volume of cells was 2.5 ml. The original container was rinsed with 0.5 ml of TE buffer and the rinse solution was added to the sporozoite solution to give a final volume of 3.0 ml. Five milliliters of proteinase K solution (0.2 M Tris, pH 8.3; .01 M EDTA; 0.4 M NaCl; 0.2% SDS; and 400 ug/ml Proteinase K (Boehringer Mannheim Biochemicals, Indianapolis, Indiana)) was then added to the sporozoite solution.

The sporozoites were incubated with the proteinase K solution at 65°C for 1 hour 8.0 ml of phenol was added to the mixture. The mixture was gently rocked on an oscillating platform for 30 minutes at room temperature. The sample was then centrifuged in a Beckman JA20 rotor at 7,000 rpm for 5 minutes at 15°C. The aqueous phase was removed with a large bore plastic pipette. The phenol interface was re-extracted by the addition of 5.0 ml of TE buffer. This solution was mixed gently by rocking, and the phases separated by centrifugation as described above. The re-extraction process was repeated. The aqueous phases were then pooled, extracted with an equal volume of chloroform ($CHCl_3$), and the pooled extractions were mixed by gently rocking. The $CHCl_3$ was separated from the aqueous phase by centrifugation and the aqueous phase was removed with a large-bore plastic pipette.

The extracted aqueous phase was adjusted to 0.3 M sodium acetate by adding a 1/10 volume of a 3.0 M sodium acetate stock solution. The DNA was precipitated from this solution by adding 2.5 volumes of cold ethanol. Gentle mixing with the cold ethanol yielded a large, viscous mass of nucleic acid that was "spooled" out of solution using a small glass rod. This material was then rinsed twice with 70% ethanol and allowed to air-dry. The dry nucleic acids were resuspended in 0.5 ml of TE buffer by rocking the solution for 16 hours at 4°C.

In order to remove any contaminating RNA from the E. tenella DNA preparation, the nucleic acids were treated with RNase A. Five-tenths milliliter of the sporozoite nucleic acid preparation was treated with 100 ug/ml RNase A (Miles Laboratories, Elkhart, Indiana) at 37°C for 30 minutes. More RNase A was added to a final concentration of 200 ug/ml, and the solution incubated at 37°C for an additional 60 minutes. An equal volume of phenol was added to the material and the mixture was gently rocked for 5 minutes. The phases were separated by centrifugation in a Brinkmann Microfuge for 1.0 minute at room temperature. The aqueous phase was removed with a large-bore pipette and extracted with an equal volume of $CHCl_3$. The aqueous phase was separated from the chloroform by centrifugation and pipetting. The aqueous phase was adjusted to 0.3 M sodium acetate by adding a 1/10 volume of a 3.0 M stock solution. The DNA was precipated from this solution by the addition of 2.5 volumes of ethanol. The ethanol precipitation yielded a large viscous pellet that was spooled from the solution and rinsed with 70% ethanol. After air drying, the DNA was resuspended in 0.5 ml of TE buffer.

Integrity of the E. tenella DNA was analyzed by electrophoresis through an agarose gel and subsequent staining of the gel with ethidium bromide. These results indicated that the DNA was of high molecular weight and was relatively free of RNA. The concentration of the DNA was determined by optical absorbance at 260 nm. This particular preparation yielded 400 ug of purified DNA.

## 3. Preparation of Small E. tenella Genomic DNA Fragments

In order to align a given fragment from the E. tenella genome in the proper reading frame for transcription and translation of the native polypeptide, it was necessary for the fragment to be adapted to the correct reading frame. The frame correctors must provide an EcoRI cohesive end for cloning into the EcoRI site of the beta-galactosidase gene by lambda gt11. The following frame correctors were used:

| NEB 1018 | 5'-GGAATTCC | n |
| NEB 1004 | 5'-CGGAATTCCG | n + 1 |
| NEB 1019 | 5'-CCGGAATTCCGG | n + 2 |

The frame correctors were obtained from New England Biolabs (Beverly, MA) and phosphorylated with polynucleotide kinase (P.L. Biochemicals) using standard phosphorylation conditions.

Because the frame correctors are blunt-ended, any restriction endonuclease that leaves a blunt end may be used to fragment the E. tenella genomic DNA. For the library described here, RsaI was selected, which recognizes the tetranucleotide sequence 5'-GTAC and cleaves between the T and A residues, generating a blunt end. Treatment of E. tenella genomic DNA with RsaI theorectically yields a cut in the DNA once every 256 base bairs on average residues, thereby generating an array of small fragments.

One hundred micrograms of E. tenella genomic DNA were cleaved using a 10-fold excess of the restriction endonuclease RsaI (New England Biolabs). The enzyme was then inactivated by heating the reaction mix to 70°C for 10 minutes. The resultant mixture was extracted with an equal volume of phenol, and residual phenol was removed by ether extractions. The reaction was made 0.3 M with sodium acetate, then 2.5 volumes of ethanol were added to precipitate the DNA. The precipitated DNA was pelleted by centrifugation, and the pellet rinsed with 70% ethanol. The RsaI fragmented DNA was resuspended in a small volume of TE buffer.

In order to protect any E. tenella genomic DNA fragments that might have internal EcoRI sites, it was necessary to modify the DNA. EcoRI methylase (New England Biolabs) was reacted with the fragmented E. tenella DNA in the presence of S-adenosyl methionine using conditions recommended by the supplier. The methylase was inactivated by phenol extraction of the reaction mix, and residual phenol was removed by ether extractions. The mix was then adjusted to 0.3 M sodium acetate, and 2.5 volumes of ethanol were added. After 30 minutes at -70°C, the precipitated DNA was pelleted by centrifugation. This DNA was resuspended in TE buffer.

The RsaI cleaved and EcoRI methylated E. tenella genomic DNA was divided into three aliquots. A different length frame corrector was added to each aliquot of small fragment E. tenella DNA. Addition of the frame corrector was such that the final concentration of corrector was 5-10 uM and in vast excess of the number of ends of fragmented genomic DNA present in each reaction. T4 DNA ligase (P.L. Biochemicals) was added to each of the three aliquots and incubated at 4°C for 16 hours. The ligation reaction was terminated by heat inactivating the mixtures at 70°C for 10 minutes. Sodium chloride to 0.1 M and excess EcoRI endonuclease were then added to the mixtures, and the reactions were incubated at 37°C for several hours. The EcoRI was heat inactivated at 70°C for 10 minutes.

Removal of excess frame correctors from the adapted insert DNA was accomplished by gel purification of the inserts. The adapted insert DNA was size-fractionated on a 2.5% agarose gel. The insert DNA was retarded by the gel matrix in such a way that adequate separation from monomer correctors was obtained after a few minutes of electrophoresis. The insert-containing region of the gel was then excised, and the DNA electroeluted from the gel.

4.  Ligation of Fragmented and Frame-Corrected DNA to lambda gt11 and In Vitro Packaging of the Recombinant DNA

Phosphatized and EcoRI-cleaved lambda gt11 DNA was mixed with frame-corrected small fragments of E. tenella. These DNAs were ligated using T4 DNA ligase (P.L. Biochemicals) overnight at 14°C. A small aliquot of each frame-corrector, ligation reaction mixture was analyzed by gel electrophoresis to monitor the ligation reaction. Each of the three reaction mixture aliquots was heated at 70°C for 5 minutes and then mixed with lambda in vitro packaging extracts. The packaging reaction was allowed to proceed for 60 minutes at room temperature at which time a drop of chloroform was added to prevent bacterial growth.

5. Amplification of the "Small Fragment" E. tenella Expression Library

Packaged phage were diluted with lambda dil and adsorbed to E. coli strain Y1088 as described by Young, R. and Davis, 2. in PNAS 80:1194 (1983). Amplification of the library on this strain ensured that the beta-galactosidase gene was not expressed; therefore, any phage containing coding sequences that might be deleterious to the host E. coli cell were not expressed and not lost from the library. Primary amplification gave libraries with the following complexity:

| corrector | 8-mer | 10-mer | 12-mer |
|---|---|---|---|
| | $1.5 \times 10^6$ | $5.1 \times 10^5$ | $1.1 \times 10^6$ |

The background level of nonrecombinants as indicated by a blue coloration on chromogenic substrate from each reaction was less than 10% of the total phage. Each of the three primary libraries had titers of from 5 x $10^9$ to 2 x $10^{10}$ phage per ml of lysate.

It was calculated, based on the number of phage in the genomic expression library, that a 50% probability of having every fragment in all three reading frames and all orientations was obtained.

B. Sporozoite cDNA Expression Library

The lambda gt11:sporozoite cDNA expression library was constructed in several steps which included purification of poly(A)$^+$ mRNA from sporozoites, construction of an initial cDNA library in lambda gt10, and transfer of the cDNA library from lambda gt10 to lambda gt11.

1. Isolation of Poly(A)$^+$ mRNA

Approximately 3 x $10^9$ E. tenella sporozoites were obtained. The sporozoites were frozen in liquid nitrogen and stored at -70°C until used. Total cellular RNA was isolated by resuspending the sporozoites in 5 ml of RNA lysis buffer (4M guanidine isothiocyanate; 0.13 M sodium acetate, pH 5.2; 0.5% Sarkosyl; 1 M beta-mercaptoethanol; 1 mM EDTA), and transferring the sporozites to a new tube. The original tube was rinsed with an additional 2 ml of RNA lysis buffer and the two solutions were combined. This mixture was vortexed 3 x 20 seconds to ensure lysis of the sporozoites.

17

The mixture was layered over a 1.25 ml cushion of 5.7 M CsCl, 50 mM sodium acetate, pH 5.2 in a SW 50.1 rotor and centrifuged for 19 hours at 35,000 rpm. The supernatant was decanted and the bottom of the tube containing the RNA pellet was removed and transferred to a 15 ml corex tube. The RNA pellet was solubilized in 3 ml of RNA lysis buffer for 1 hour at 60°C. After removal of the tube bottom, 6 ml of distilled water was added, followed by the addition of 18 ml of ethanol. The RNA was allowed to precipitate overnight at -20°C, and the precipitate was collected by centrifugation at 10,000 rpm for 10 minutes in a JA-10 rotor (Beckman Instruments). The RNA was resuspended in 4 ml of 0.3 M sodium acetate, pH 5.2. The corex tube was washed with an additional 0.5 ml of 0.3 M sodium acetate pH 5.2. The RNA and wash solutions were combined and the RNA was precipitated by adding 10 ml of ethanol and letting the mixture remain at -20°C overnight. The RNA was pelleted by centrifugation as before and the pellet was washed with ethanol, vacuum dried and resuspended in 400 microliters of distilled water for a final volume of 500 microliters. The yield of total cellular RNA from the $3 \times 10^9$ sporozoites was approximately 800 micrograms.

Poly(A)$^+$ mRNA was isolated by oligo (dT)-cellulose chromatrography using standard techniques as described in Edmonds, M. et al. PNAS 68:1336(1971). The total cellular RNA suspended in 1.2 ml distilled water was mixed with 300 microliters of 5X binding buffer (50 mM Tris-HCl, pH 7.5; 2.5 M NaCl) and passed through a 1 ml column of oligo (dT)-cellulose (Type III, Collaborative Research, Lexington, MA). The column was washed with 15 ml of 1X binding buffer (10 mM Tris-CHl, pH 7.5; 0.25 M NaCl) and poly(A)$^+$ mRNA was eluted by the addition of 3 ml of 10 mM Tris-HCl, pH 7.5. Fractions containing poly(A)$^+$ mRNA were pooled, diluted with one-quarter volume of 5X binding buffer and passed through the rejuvenated oligo (dT)-cellulose column. Fractions of the eluate containing poly (A)$^+$ mRNA were pooled, mixed with one-tenth volume of 3 M sodium acetate, pH 5.2, and precipitated with ethanol overnight at -20°C. The RNA was collected by centrifugation in a SW 41 rotor for 30 minutes at 25,000 rpm. The RNA pellet was washed with ethanol, vacuum dried and resuspended in 40 microliters of distilled water. Approximately 13 micrograms of poly(A)$^+$ mRNA were recovered.

2. Construction of the Lambda gt10:Sporozoite cDNA Library

Two micrograms of poly(A)$^+$ mRNA were converted into cDNA using AMV reverse transcriptase (Life Sciences, St. Petersburg, FL). The reaction was primed with oligo (dT$_{12-18}$) (Collaborative Research, Lexington, MA). A small molar amount of $^{32}$P-dATP was included in the reaction to provide a tracer for subsequent steps. After 60 minutes at 42°C, the cDNA was separated from the unincorporated nucleotides and other small molecules by passage through a 1 ml column of Sephacryl S-300 using 20 mM Tris-HCl pH 8.0, 100 mM NaCl, 1 mM EDTA. Fractions containing cDNA were identified by scintillation count, pooled, and recovered by ethanol precipitation. A string of dG residues were added to the 3' ends of the cDNA using terminal deoxynucleotide transferase (PL Biochemicals). At the completion of this reaction, RNase A and oligo (dC$_{12-18}$) (Collaborative Research) were added directly to the mixture which was then put in a boiling water bath for 45 seconds, cooled to room temperature and then put on ice.

Second strand cDNA synthesis was performed by adding all four deoxynucleotides and E. coli DNA polymerase I to the reaction mixture and incubating the mixture at 15°C for 18 hours. Double-stranded cDNA was then treated with EcoRI endonuclease. After phenol and ether extractions, the cDNA was passed through a 1 ml Sephacryl S-300 column as described above. Fractions of eluate containing cDNA were pooled and the cDNA recovered by ethanol precipitation.

The cDNA was ligated overnight to phosphorylated EcoRI linkers (New England Biolabs, Beverly, MA) and treated with several hundred units of EcoRI to digest excess linkers. The mixture was then passed through a 1 ml Sephacryl S-300 column and the fractions of eluate containing cDNA were pooled and precipitated with ethanol. The double-stranded cDNA was resuspended in a small volume of water and stored at -20°C.

To clone the cDNA into the unique EcoRI site of lambda gt10 as described by Huyhn, Young, and Davis in DNA Cloning Techniques, A Practical Approach, Glover, D.M. [ed.], IRL Press, Oxford, in press, lambda gt10 DNA was digested to completion with EcoRI, ligated to cDNA overnight at 16°C and packaged in vitro. Aliquots were plated on E. coli C600 and C600 HflA to determine the number of total phage and the number of recombinant phage containing cDNA inserts. From serval ligations and packagings, a total of $2 \times 10^7$ phage were recovered, 15-18% or approximately $3 \times 10^6$ of which contained cDNA inserts.

The phage in this library were amplified on E. coli C600 HflA to eliminate non-recombinant phage. The phage were plated at a density of $5 \times 10^4$ per 150 mm plate, incubated at 37°C until plaques appeared and overlaid with 12.5 ml of 10 mM Tris-HCl, pH 7.4; 10 mM MgSO$_4$ per plate. A total of $3.2 \times 10^{12}$ phage were recovered from the amplified library. Over 99% of the recovered phage were recombinants.

### 3. Moving the Lambda gt10:Sporozoite cDNA Library Into the Expression Phage Lambda gt11

DNA was obtained from the amplified lambda gt10:sporozoite cDNA library by growing plate lysates of the phage. Sixty-four 150 mm plates were seeded with 1 x 10$^5$ phage per plate and 0.7 ml of an overnight culture of E. coli C600 which had been resuspended in one-half volume of 10 mM MgSO$_4$. After lysis, each plate was overlaid with 12.5 ml of 10 mM Tris-HCl pH 7.4, 10 mM MgSO$_4$ and stored overnight at 4°C. Phage were purified from the liquid overlay by conventional procedures using polyethylene glycol precipitation and CsCl banding in a step gradient with a final banding in a CsCl equilibrium gradient. The DNA was obtained from the phage by extraction with formamide as described by Davis, R.W., et al. in Advanced Bacterial Genetics (Cold Spring Harbor Laboratory, New York, 1980). A total of 1.4 mg of phage DNA was recovered.

The cDNA inserts were obtained from this phage DNA by digestion with EcoRI endonuclease and size-fractionated on sucrose gradients. 200 micrograms of phage DNA were digested with 1,000 units of EcoRI, phenol extracted and ethanol precipitated. Approxiamtely 5% of the DNA was labeled at the EcoRI sites with $^{32}$P-dATP and the Klenow fragment of E. coli DNA polymerase I to serve as tracer. The DNA was suspended in a 100 mM NaCl solution, heated to 42°C for 5 minutes to anneal the phage arms, and size-fractionated on a 10-40% sucrose gradient made up in 20 mM Tris-HCl, pH 8.0, 5 mM EDTA, and 1 M NaCl. The sucrose gradient was centrifuged in an SW 41 rotor at 15°C for 24 hours at 30,000 rpm. Fractions containing cDNA were identified by scintillation count, pooled, dialyzed against 10 mM Tris-HCl, pH 8.0, 1 mM EDTA and precipitated with ethanol. The resuspended cDNA was centrifuged through a second 10-40% sucrose gradient using identical procedures except that fractions of the gradient were analyzed by gel electrophoresis and autoradiography to determine cDNA size. Fractions containing cDNA larger than 500 bp were pooled, dialyzed against 10 mM Tris-HCl, pH 8.0, 1 mM EDTA, ethanol precipitated and resuspended in a small volume of water.

Purified cDNA was ligated to EcoRI-digested and phosphatized (using calf intestinal phosphatase) lambda gt11 DNA, and packaged in vitro. Aliquots of the packagings were plated on E. coli Y1088 (using the method described by Young and Davis in Science 222: 778-782 (1983)) in the presence of X-GAL and IPTG in order to determine the total number of phage and the number of recombinant phage. Total phage recovered from several ligations and packagings were 1 x 10$^6$, of which greater than 90% were recombinants.

A portion of this library (7 x 10$^5$ phage) was amplified on plates using E. coli Y1088. The phage were plated at a density of 4 x 10$^4$ per 150 mm plate. The final amplified library had a titer of 2.2 x 10$^{10}$ phage per ml. The amplified lambda gt11:sporozoite cDNA library was used as a source of phage for screening with antibodies and other probes as described below.

### 4. Determination of cDNA Size

DNA from positive phage was purified from plate lysates using similar procedures as described above for obtaining DNA from the lambda gt10:sporozoite cDNA library except that E. coli Y1088 was used as the host and the number of phage plated per plate was 3 x 10$^6$. cDNA sizes were determined by digesting 0.5 micrograms of phage DNA with EcoRI and analyzing the products by agarose gel electrophoresis. Commercially available lambda DNA digested with HindIII and ØX 174 DNA digested with HaeIII served as size markers.

### II. GENERAL METHODOLOGY

#### A. Antibody Screening of the Lambda GT11:Tenella Expression Libraries

The lambda gt11:tenella expression libraries (cDNA or genomic) were plated at densities of 1 x 10$^4$ through 2 x 10$^5$ phage per 150 mm plate using E. coli Y1090 as host as described by Young and Davis in Science 222:778-782 (1983). Plates were incubated at 37°C or 42°C for 4 hour, then overlaid with nitrocellulose filters (BA-85, Schleicher and Schuell) that had been soaked in 10 mM IPTG and air-dried. After incubating overnight at 37°C, filters were batch-washed 3 x 10 minutes in TBS (10 mM Tris-HCl, pH 8.0; 150 mM NaCl). Non-specific protein binding sites on the filters were blocked by incubating filters for 60 minutes in TBS + 2% bovine serum albumin (Fraction V, Miles Laboratories, Elkhart, IN). The filters were then incubated individually or in pairs for 2 hour with 10-20 ml of primary antibody (e.g., immune chicken serum, hyperimmune rabbit anti-sporozoite serum, mouse monoclonal anti-sporozoite antibodies) typically diluted 1:200 (1:500 for monoclonals) with TBS to which had been added 2% bovine serum albumin (BSA). The filters were washed 3 x 10 min with TBS containing 0.1% NP-40 (obtained from Sigma), then incubated singly or in pairs for 60 min with 10-20 ml solution of a second antibody (e.g., peroxidase-conjugated goat anti-rabbit IgG, Cappel Laboratories) diluted 1:500 in TBS + 2% bovine serum albumin. Filters were batch-washed 3 x 10 min in TBS and stained in a solution comprising 200 ml TBS, 2.5 ml hydrogen peroxide and 40 ml of a 3 mg/ml solution of 4-chloro-1-napthol in methanol. Staining was quenched by removing the filters to water. Positive staining plaques were subjected to several rounds of rescreening with antibody as described above until pure.

### B. Elution Of Antibodies Bound To Proteins Immobilized On Nitrocellulose Membranes

When proteins are immobilized on nitrocellulose membranes such as Western blot transfers of proteins separated on polyacrylimide gels or replicas of phage plaques taken from agar plates, it is possible to bind antibodies which specifically recognize the immobilized proteins. Antibodies which do not specifically bind to the immobilized proteins remain in solution and can be easily washed away, leaving behind only those specifically bound.

The bound antibodies can be eluted by rinsing the filters in a low pH buffer (5 mM glycine, pH 2.3, 0.5 M NaCl, 0.5% Tween 20, 0.01% BSA), which dissociates the antibody-antigen complex. If the eluted antibodies are immediately neutralized,i.e., using a 50 mM Tris-HCl, final concentration, they retain full activity and can be used for a variety of analytical purposes. Eluted antibodies have been used to screen the recombinant expression libraries and determine the sporozoite protein that corresponds to a given insert clone using the methods that follow.

### 1. Screening Recombinant Expression Libraries With Eluted Antibodies

Antibodies eluted from specific bands on a Western blot were used to screen recombinant expression libraries in order to find recombinant clones expressing antigens corresponding to the protein band on the Western blot. This technique allowed isolation of a number of the insert clones.

In order to obtain enough antibodies to screen the library, a Western blot was prepared from SDS-polyacrylimide gel consisting of a single wide lane of site functional E. tenella sporozite proteins. After non-specific protein binding sites on the nitrocellulose were blocked using 2% BSA in TBS, 1 hour at room temperature, the blot was washed 3 x 10 minutes in TBS at room temperature. Polyclonal rabbit anti-sporozoite serum diluted 1:50 in TBS + 2% BSA was bound to the blot overnight at room temperature. Unbound antibodies were removed by washing in TBS + 0.2% Tween 20 (Sigma).

A thin strip was cut from each side of the blot for staining in order to indicate the positions of bound antibodies on the central part of the blot. Second antibody, peroxidase-conjugated goat antirabbit IgG (Cappell Laboratories); 1:500 in TBS + 2% BSA was bound to the strips. The strips were washed 2 x 10 minutes in TBS + 0.2% Tween 20 followed by a 10 minute wash in TBS) and subsequently stained for 20 minutes at room temperature in 50 ml TBS + 10 ml 4-chloro-1-naphtol (3 mg/ml in methanol) + 0.6 ml 3% $H_2O_2$.

The stained strips were lined up with the main body of the blot, and strips were cut in the main blot which corresponded to the desired bands on the side strips. The main blot strips were placed in long polypropylene sealable test tubes. A small volume ( 0.5 ml) of elution buffer (5 mM glycine, pH 2.3; 0.5 M NaCl, 0.5% Tween 20; 0.01% BSA) was added to each tube and the tubes were rocked end to end to allow the buffer to wash along the filter strip. After 30 seconds, the buffer was removed and immediately neutralized with a 1/20 volume of 1 M Tris-HCl, pH 7.4. The elution was repeated two more times for 30 seconds each with fresh elution buffer. All three washes were pooled. The eluted antibodies were diluted in TBS + 0.2% Tween 20 to an appropriate volume for screening the recombinant libraries, as described in Section IIA, infra.

### 2. Determination of Sporozoite Protein Corresponding to Insert Clone

Antibodies eluted from plaque replicas of a purified recombinant clone were used to determine which sporozite protein corresponded to that clone. By eluting antibodies bound to plaque replicas of the recombinant clones and using those antibodies to probe Western blots of sporozite proteins, it was possible to determine which protein was encoded in the recombinant clone.

Five thousand to ten thousand plaques from a single purified recombinant clone were plated on a 100 mm plate, and a plaque lift was made as described in Section IIA, supra. The lift filters were washed 3 x 10 minutes in TBS and non-specific protein binding was blocked by incubation in TBS + 10% BSA for 1 hour. The filters were washed 3 x 10 minutes in TBS. A strip 5 mm wide was cut from the center of the filter disc. Polyclonal anti-sporozite serum was bound to the strip, washed and eluted as described for the Western blot elutions in Section III.A.1 above. The eluted antibodies were then used to probe a Western blot of sporozite proteins.

### III. CONSTRUCTION OF THE LAMBDA EMBL3::EIMERIA TENELLA GENOMIC LIBRARY

The construction of a lambda EMBL3::E. tenella genomic DNA library was accomplished in five steps. First, in vitro bacteriophage lambda packaging extracts were prepared for efficient cloning of the recombinant phage. Second, lambda EMBL3 vector DNA was prepared. Third, preparation of fragmented E. tenella genomic DNA was accomplished by partial restriction endonuclease cleavage and size selection. Fourth, the E. tenella DNA was ligated to the lambda EMBL3 cloning vector and packaged in vitro. Finally, the in vitro packaged recombinant phage were amplified on a recombinant selective host strain.

#### A. Lambda in vitro Packaging Reagents

Bacteriophage lambda in vitro packaging extracts from E. coli lysogens BSB2690 (Dam prehead donor) and BHB 2688 (Eam packaging protein donor) were prepared and used as described bv Sternberg et al. in Gene 1:255 (1977). In some experiments, lambda in vitro packaging extracts were purchased from Boehringer Mannheim and used according to the manufacturer's directions.

The strains were grown separately, induced by temperature shift, and concentrated by centrifugation. Cells were broken by sonication (prehead donor) and by lysozyme treatment followed by rapid freezing and thawing (packaging protein donor). After centrifugation to remove cellular debris and DNA, the extracts were frozen in small aliquots. Packaging was then accomplished by adding lambda DNA to a mixture of both extracts followed by incubation at room temperature. Using various combinations of extracts, a packaging efficiency of $4 \times 10^7$ plaque-forming units per microgram of lambda DNA was obtained. This efficiency was found to be adequate for library construction.

#### B. Preparation of Lambda EMBL3 Vector DNA

Lambda EMBL3 is a replacement vector with a nominal cloning capacity of 9 to 20 kb (77 to 105%) of wild-type lambda. A lysate of lambda EMBL3 was obtained from Dr. Matt Scott (MCDB, University of Colorado). EMBL3 allows for a double selection against non-recombinant phages. First, there is a genetic selection against religated vector by amplifying the library on a P2 lysogenic host E. coli (Q359), as described by Karn et al. in Proc. Nat. Acad. Sci. 77:5172-5176 (1980). E. coli Q359 is non-permissive for intact spi$^+$ EMBL3 phage. Second, EMBL3 allows a biochemical selection against the ligation event reinserting the phage middle fragment into the phage by double cleavage with BamHI and EcoRI and removal to the small linker fragment by isopropanol precipitation.

Lambda EMBL3 phage stock was prepared essentially as described by Davis, R.W., et al. in Advanced Bacterial Genetics (Cold Spring Harbor Laboratory, New York, 1980) using the plate lysis method. E. coli Q358 cells were grown overnight in L-broth + 0.2% maltose and harvested by centrifugation. The pelleted Q358 were resuspended in 10 mM $MgCl_2$ and lambda EMBL3 phages were added to the cells at a multiplicity of infection of 1. The phage were allowed to adsorb to the cells for 30 minutes at room temperature, mixed with 7.5 ml of HT soft agarose and plated on a 150 mm HT plate.

Ten plates were prepared in this manner and the plates were incubated at 37°C for 5 hours, at which time confluent lysis had occurred. The plates were placed at 4°C, and each was overlayed with 12.5 ml of cold lambda dil (10 mM $MgCl_2$; 10 mM Tris-HCl, pH 7.4; 1 mM EDTA) and incubated overnight. The overlay solutions were then pooled and centrifuged in a Beckman JA-20 rotor at 10,000 rpm for 10 minutes at 4°C to remove cellular debris.

The phage were then pelleted from the cleared lysate by centrifugation at 20,000 rpm in a JA-20 rotor for 3 hours at 4°C. The phage pellet was resuspended in lambda dil and layered atop a CsCl gradient prepared in an SW 41 (Beckman Instruments) centrifuge tube. The gradient was spun at 35,000 rpm for 60 minutes at 15°C. The phage band was removed from the gradient by extraction with a syringe and then mixed with an equal volume of 1.7 g/ml CsCl. This solution was dispensed underneath another CsCl gradient in an SW 41 tube and then centrifuged at 35,000 rpm for 60 minutes at 15°C. The resultant phage band was removed with a syringe and the contents placed into dialysis tubing. The phage were dialyzed against 50% formamide, 0.1 M Tris-HCl (pH 8.5) for 24 hours at room temperature to release the phage DNA from the intact bacteriophage. The released DNA was dialyzed versus 0.1 M NaCl, 50 mM Tris-HCl (pH 7.5), and 1 mM EDTA for 16 hours with four changes of buffer in order to remove the formamide. The purified DNA was then removed from the dialysis membrane and stored at 4°C in a test tube. The DNA concentration of this preparation was determined by absorbance at 260 nm and gave a yield of 120 mg/ml.

Purified lambda EMBL3 DNA was cleaved with the restriction endonuclease BamHI. After digestion, the BamHI enzyme was inactivated by adding EDTA and heating the reaction mix at 70°C for 10 minutes. The DNA solution was adjusted to 0.3 M sodium acetate and 2.5 volumes of ethanol were added to precipitate the DNA. The DNA was recovered by centrifugation, and the pellet was resuspended in TE buffer as described in Section I.A1 (page 37), supra.

The BamHI-cleaved DNA was then cleaved with EcoRI at 37°C for 60 minutes. The EcoRI was inactivated with EDTA, and the resultant mixture was then extracted with an equal volume of phenol. Residual phenol was removed by ether extraction of the aqueous phase. The doubly-cleaved DNA was then separated from the small EcoRI-BamHI polylinker sequences by precipitation of the mixture with isopropanol. The resultant pellet was resuspended in buffer and reprecipitated with ethanol. The DNA was then resuspended in 1/10 TE buffer and used for library construction.

### C. Preparation of Fragmented E. tenella Sporozoite Genomic DNA

The bacteriophage lambda EMBL3 is designed to accept recombinant DNA inserts with cohesive termini that are compatible with termini generated by cleavage with BamHI, i.e., termini with end structures of 5'-GATC. Sau3A is a restriction endonuclease that recognizes the tetranucleotide sequence 5'-GATC in DNA and cleaves on the 5' side of the G residue, generating a cohesive end appropriate for the EMBL3 BamHI cloning site.

E. tenella sporozite DNA prepared as described above was used in small-scale digests with Sau3A to empirically determine the reaction conditions that would generate approximately 20 kb partial digestion products. These conditions were then scaled up and 100 ug of sporozoite DNA was partially digested with Sau3A, and the enzyme was immediately inactivated by phenol extraction of the reaction mix. After the phases were separated by centrifugation, the aqueous phase was removed with a large bore pipetter and residual phenol removed by chloroform extraction. The sample was adjusted to 0.3 M Sodium Acetate (NaOAc) with a concentrated stock solution, and 2.5 volumes of ethanol were added to precipitate the DNA. The precipitated DNA was concentrated by centrifugation and the pellet resuspended in TE buffer. This DNA was layered on top of a 10-40% sucrose gradient preformed in an SW28 (Beckman) centrifuge tube. The gradient was centrifuged at 26,000 rpm at 20°C for 24 hour.

The gradient was fractionated by pumping liquid from the bottom of the tube through a glass capillary in 0.5 ml aliquots. The fractions were analyzed by gel electrophoresis to determine which aliquots harbored the appropriately sized fragments (15-20 kb). Those fractions were pooled, placed into a dialysis membrane and dialyzed against TE buffer. After dialysis, the sample was extracted 3 times with an equal volume of butanol to concentrate the sample. The salt concentration was adjusted to 0.3 M. with sodium acetate, and the DNA precipitated with 2.5 volumes of ethanol. The DNA was concentrated by centrifugation, rinsed with 70% ethanol, and resuspended in a small volume of TE buffer. This DNA was analyzed by gel electrophoresis to confirm that it was of appropriate size (15-20 kb). The yield was 500 ng.

### D. Ligation of Fragmented E. tenella DNA to EMBL3 Vector and in vitro Packaging of the Recombinant DNA

Lambda EMBL3 DNA cleaved with BamHI and EcoRI and purified as described above was mixed at a 2:1 mass ratio with sized E. tenella genomic DNA prepared by partial Sau3A digestion. These DNAs were ligated with T4 DNA ligase overnight at 14°C. A small aliquot was analyzed by gel electrophoresis to monitor the ligation reaction. After brief incubation at 70°C, the ligation reaction was mixed with the in vitro packaging extracts and incubated for 60 minutes at room temperature. Chloroform was then added to prevent bacterial growth and the phages were ready for amplification.

E. Amplification of the E. tenella Library

Packaged phage were diluted with lambda dil and adsorbed to fresh E. coli Q359 plating cells. The cells were mixed with NZC soft agarose and plated on NZCYM plates (described by Maniatis, T. et al., in Molecular Cloning, Cold Spring Harbor Laboratory, New York, (1982). Plates were incubated at 37°C for 10 hours. The complexity of the library was determined by counting plaques in representative small regions of the plates. The total yield of recombinants was 150,000. The recombinant phage were eluted from the primary amplification plate by overlayering with lambda dil. The titer of the amplified library was $3 \times 10^7$ plaque forming units (pfu) per ml. The background of nonrecombinants in the library was determined by differential plating of the amplified library on E. coli Q359. which allowed only recombinant phages to form plaques on E. coli Q358 which allows both recombinant and nonrecombinant phages to form plaques, and on E. coli KRO which allows only nonrecombinant phages to form plaques. (Karn, J.M. et al. 1980 PNAS 77; Frischauf, A. et al. (1983), J. Mol. Biol. 170) E. coli Q358 and lambda EMBL3 from Dr. D. Hirsh, E. coli Q359 and KRO from Dr. M. Scott, both of MCDB University of Colorado. The background of nonrecombinant phages in the amplified library was less than 0.01% of the plaques. Analysis of randomly selected recombinant phages indicated that the average size of the E. tenella DNA inserts was 17 kb.

## IV. PLASMID VECTORS FOR EXPRESSION OF FUSION PROTEINS

A number of antigenically reactive E. tenella recombinant phage clones were identified in the sporozite cDNA expression library and the "small fragment" E. tenella genomic expression library. Since the lambda gt11 lysogens appeared to make a limited quantity of fusion protein, a plasmid expression vector was constructed that would produce the fusion proteins in milligram quantities.

### A. pSEV4

Plasmid pLG2 was obtained from Dr. L. Guarente (MIT) (Guarente, L., in Cell, 20:543-553 (1980)). This vector is a pBR322 derivative which, like lambda g+11, has lac operator and promoter sequences in addition to a wild-type beta-galactosidase gene containing a single EcoRI site near the 3' end of the gene. In addition, pLG2 contains the lac repressor gene. Moving E. tenella DNA inserts from lambda gt11 into the EcoRI site of this vector yields a fusion protein identical to that initially identified in the phage.

Plasmid pLG2 was modified to remove an extra EcoRI site prior to its use for expression. Plasmid pLG2 was partially digested with EcoRI restriction endonuclease to linearize the plasmid. The plasmid DNA was then displayed on a preparative agarose gel and the linear-sized DNA band was eluted from the gel. The eluted DNA was precipitated by making the solution 0.3 M with sodium acetate and adding 2.5 volumes of ethanol. The DNA was pelleted by centrifugation and the pellet was resuspended in TE buffer. The Klenow fragment of E. coli DNA Polymerase I was mixed with the DNA in the presence of dATP and dTTP to fill in the EcoRI cohesive ends. After heat inactivation at 70°C for 10 minutes, T4 DNA ligase (P.L. Biochemicals) was added and the mixture was incubated at 4°C for 16 hours. The ligated DNA was then used to transform E. coli AMA1004. (Casadaban, M., et al., in Methods in Enzymology 100:293 (1983).

Transformants were selected on ampicillin plates in the presence of a chromagenic substrate for beta-galactosidase activity (X-GAL). Transformants with beta-galactosidase activity were screened by cleaving the DNA with EcoRI. A plasmid, pSEV4, that had only a single EcoRI site near the carboxyl terminus of the beta-galactosidase gene was identified from the transformants and characterized.

Plasmid pSEV4 has a unique EcoRI site near the carboxyl terminus of the beta-galactosidase gene. Plasmid pSEV4 contains the wild-type lac operator, promoter and repressor in addition to the beta-galactosidase gene. Upon induction with IPTG for 60 minutes, beta-galactosidase activity was increased by 300-fold. Uninduced cells containing pSEV4 produced approximately 1000 units/mg of total cellular protein, whereas IPTGinduced cells gave approximately 300,000 units/mg of total cell protein. Protein gel analysis of induced and uninduced cells also showed the overproduction of beta-galactosidase by induced cells. This new plasmid, pSEV4 (see Fig. 10), has been used to express a number of E. tenella antigens as fusion proteins.

### B. pSEV6

A successor to pSEV4 was constructed to allow polarized "cassette" subcloning of DNA inserts from lambda gt11 directly into a plasmid expression vector. Because EcoRI inserts could be subcloned in either orientation in pSEV4, each pSEV4 subclone for its antigenic reaction. Polarized subcloning using pSEV6 obviates the need for this extra analysis.

Extensive mapping of pSEV4 located five restriction endonuclease sites in the lac operon 5' to the beta-galactosidase gene's unique EcoRI site. Three of these sites are unique and two were made unique by deletion of the superfluous DNA between the lacI gene and the pBR322-derived amp$^r$ gene. Only one useful restriction site was found 3' to the EcoRI site, the Nco1 site, therefore, additional restriction enzyme sites were inserted in this region using a chemically synthesized polylinker.

The construction of pSEV6 was done in two steps. First, pSEV4 was shortened by approximately 5,700 bp to eliminate superfluous DNA. Plasmid pSEV4 was cleaved with SphI restriction endonuclease, and the enzyme was inactivated by heating at 70°C for 10 minutes. The DNA was then partially digested with AatII and the resulting digest was displayed by electrophoresis on a preparative agarose gel. The 7,620 bp fragment was excised from the gel and electroluted.

The electroluted DNA was precipitated from a 0.3 M sodium acetate solution by adding 2.5 volumes of ethanol and incubating at -70°C for 30 minutes. The DNA was concentrated by pelleting in a Brinkman micro-centrifuge for 15 minutes and the pellet was resuspended in TE buffer. T4 DNA polymerase (New England Biolabs) was added to blunt-end the cohesive ends generated by the AatII digest. After heat inactivation of the T4 DNA polymerase, T4 DNA ligase (P.L. Biochemicals) was added to ligate the blunt ends of the DNA fragment. The ligated DNA was used to transform competent AMA1004 E. coli cells. Lac$^+$ transformants were screened for the 7,620 bp plasmid. One plasmid, pSEV5, was identified and characterized as having the appropriate structure.

DNA from pSEV5 was purified by standard methods, and subsequently cleaved with the restriction endonuclease NcoI, which cleaved at a unique site 3' of the beta-galactosidase gene. An oligonucleotide adapter molecule that would regenerate the NcoI site and which also contained BglII and KpnI sites was chemically synthesized. This oligonucleotide was ligated to the NcoI cleaved pSEV5 with T4 DNA ligase (P.L. Biochemicals). The ligated DNA was used to transform competent E. coli AMA1004 cells. DNA from the resulting lac$^+$ transformants was screened for the presence of the unique KpnI and NcoI sites. A plasmid was identified from this screening with all of the designed sequences. This plasmid, pSEV6 (see Fig. 11), has been used for the expression of various of the antigenically reactive fusion proteins.

## V. PURIFICATION OF THE CLONED ANTIGENS FOR ANIMAL TESTING

The beta-galactosidase::E. tenella antigen fusion proteins have been purified either by use of a substrate analog affinity column for beta-galactosidase or by classical methods of protein purification.

### A. Preparation of Extracts

Two liters of Luria broth, pH 7.5, described by Maniatis, T. et al., in Molecular Cloning (Cold Spring Harbor Laboratory, Cold Spring Harbor, New York, 1982) containing 50 ug/mL of ampicillin were inoculated with 10-20 ml of an overnight culture of E. coli AMA1004 containing one of the recombinant plasmids. The cells were allowed to grow at 37°C to mid-log phase (A$_{600}$ = 0.2). Isopropyl-thiogalactoside (IPTG) was added to a final concentration of 1 mM to induce formation of the fusion protein. The cells were allowed to grow out for 2 hours, and then harvested by centrifugation at 5000 x G for 15 minutes at 4°C. All subsequent operations were carried out at 4°C.

The cells were resuspended in 20 mL of breaking buffer (50 mM Tris-HCl, pH 7.5; 250 mM NaCl; 10 mM MgCl$_2$; 5% glycerol; 1 mM phenymethyl sulfonyl fluoride (PMSF)) at 4°C and centrifuged again at 5000 x G for 15 minutes. The cells were again suspended in 20-40 mL of breaking buffer (See Section VA (page 67), supra.). The cells could be frozen at this point and stored at -20°C if desired.

The unfrozen or thawed cells were broken with two passes through a French pressure cell (Aminco) at 20,000 psi. Cell debris was removed by centrifugation at 30,000 x G for 30 minutes. Further clarification of the extract could be obtained at this point by ultracentrifugation at 100,000 x G for 30 minutes. The fusion protein was then precipitated by the addition of ammonium sulfate to a final concentration of 20 to 40% saturation. The optimal concentration of ammonium sulfate required for precipitation of the fusion protein varies with the individual protein and must be determined experimentally using, for example, procedures set forth in Heppel, L. in Methods in Enzymology, 1:570-576 (1955).

The precipitate solution was stirred for one hour and the precipitate was removed by centrifugation at 30,000 x G for 15 minutes. The pellet was redissolved in 10 to 15 ml of starting buffer (50 mM Tris-HCl, pH 7.5; 250 mM NaCl; 10 mM MgCl$_2$; 1 mM dithiothreitol (DTT); 0.1% Triton X-100), and then dialyzed overnight against 500 ml of starting buffer.

## 2. Affinity Purification Procedure

The use of a beta-galactosidase affinity column is based on the methods described by Steers and Cuatrecasas in Methods in Enzymology, 34:350-355 (1974). Affinity resin (p-amino-phenyl-beta-D-Thiogalactopyranoside-agarose, obtained from Sigma) was packed into a 1.5 cm diameter by 15 cm column. The column was washed with 10 column volumes of starting buffer of 50 mM Tris-HCl (pH 7.5), 250 mM NaCl, 10 mM $MgCl_2$, and 1.0 mM dithiothreitol (DTT) and 0.1% Triton-X100 before use. The column can be regenerated after use by washing extensively with elution buffer 0.1 M sodium borate (pH 10), 250 mM NaCl, 1 mM DTT or by washing with 6M guanidine hydrochloride in 50 mM Tris-HCl, pH 7.5. After washing, the column is reequilibrated with 10 column volumes of starting buffer.

For affinity chromatography, dialyzed material was applied to the pre-equilibrated affinity column at a flow rate of about 0.2 ml/min. After the sample was applied to the column, the column was washed with 15 ml of starting buffer at the same flow rate, then with 30 ml of starting buffer at about 0.5 ml/min followed with 180 ml starting buffer at about 1 ml/min. Finally, the column was washed with 120 ml of starting buffer without Triton X-100 at the same flow rate.

The absorbed protein was eluted with 0.1 M sodium borate, pH 10.0; 250 mM NaCl; and 1 mM DTT using 120 ml at a flow rate of about 1 ml/min. The peak-protein containing fractions are pooled and could be concentrated if desired to about 10 ml using an Amicon ultrafiltration device (Model 8050) containing an YM-30 membrane.

## 3. Ultracentrifuge Purification

An alternative purification useful for some of the fusion proteins (e.g., pSEV4::CH2-13) is accomplished by obtaining dialyzed protein as set forth above. The dialyzed material is subjected to ultracentrifugation at 100,000 x G for 30 minutes. The pellet containing the bulk of the fusion protein is redissolved in a small volume of dialysis buffer 50 mM Tris-HCl (pH 7.5), 250 mM NaCl, 10 mM $MgCl_2$ and 1.0 mM DTT and 0.1% Triton-X100. This method yields material that is not as pure as that generated by the affinity column when judged by SDS-polyacrylamide electrophoresis.

## 4. Analysis

The purified materials obtained by these methods were analyzed for protein by the Bio-Rad (Richmond, CA 94804) protein method described by the manufacturers. They were also subjected to analysis by SDS-polyacrylamide electrophoresis. These gels are visualized either by protein staining or by Western blot analysis. Protein staining was typically done with either the silver stain method as described by Wray, W.P. et al. in Anal. Biochem. 118:197-203 (1981) or the Bio-Rad protein stain method. Western blot analysis is carried out as described by Renart, J. et al., in PNAS (USA) 76:3116 (1979).

The Western blot analysis involves electrophoretic transfer of the resolved protein bands to nitrocellulose, blocking the nitrocellulose paper with BSA, probing with a specific antibody (either anti-betagalactosidase or anti-sporozite sera). After washing, the blots are probed with the appropriate peroxidase conjugated second antibody, followed by color development using the peroxidase catalyzed reaction.

## VI. PROCEDURES FOR OBTAINING THE ENTIRE E. tenella GENE ENCODED BY A cDNA OR SMALL GENOMIC FRAGMENT

Many of the recombinant E. tenella clones that are reactive with serum from immune animals probably contain only a portion of the entire coding region for a particular polypeptide. The small fragment genomic expression clones were fragmented intentionally so that the antigenic polypeptide is on the order of 100 or so amino acids in length. Most of the "small fragment" clones encode polypeptides larger than 100 amino acids. The E. tenella sporozoite cDNA clones may encode only a portion of the gene because the mRNA used to obtain the clone was truncated. The cDNA may have been shortened because the mRNA that was isolated was not full length, or the full-length cDNA contained a naturally occurring EcoRI restriction endonuclease site. In the latter situation, the lambda gt11 cDNA expression library was obtained by moving the cDNA from lambda gt10 into gt11 by cleaving the cloned cDNA with EcoRI. In some cases, it may be important to clone the entire coding sequence of a given antigenic E. tenella polypeptide in order to maximuze the immune response, or modulate the response. Consequently, large E. tenella genomic lambda EMBL3 recombinant phages have been isolated that encode the entire gene.

Briefly, the antigenic E. tenella cDNA or "small fragment" E. tenella genomic fragment is subcloned into the EcoRI site of the E. coli plasmid pBR325, described by Prentki,P., et al.[1981] in Gene 14, 289, specifically incorporated herein by reference. Plasmid DNA is then prepared from the chloramphenicol resistant transformant. The DNA is then 'nick translated' using E. coli DNA polymerase to incorporate $^{32}$P-labeled deoxynucleotides into the DNA. This labeled DNA is then used as a radioactive probe to select an homologus E. tenella sequence from the large genomic recombinant lambda EMBL3 library. Phage selected from the recombinant library by this method are plaque purified, DNA is prepared, and the E. tenella specific insert is mapped with various restriction endonucleases. The resulting map is compared with a similar map derived from the initial clone to confirm the identity of the large genomic phage.

Because of the presence of introns in some of the genes that have been characterized, it is not certain that the entire coding region is contained within the large genomic phage. However, given the deposited phage, the restriction map, and the E. tenella large genomic library in lambda EMBL3, it is possible for anyone skilled in the art to obtain the entire gene. This is done by "walking" along the E. tenella genome by isolating phage that contain flanking E. tenella genomic DNA using the method described by Bender, W. et al., in JMB 168:17-33 (1983), specifically incorporated herein by reference.

## VII. PROCEDURES FOR ISOLATING LAMBDA EML3 E. tenella GENOMIC PHAGE THAT CONTAIN THE REPEATING TRINUCLEOTIDE CAG

E. tenella genomic and cDNA lambda gt11 expression phage that encode polypeptides F, G and M contain repetitions of the trinucleotide CAG, and consequently these phage all cross-hybridize with each other at the DNA level even though they encode different polypeptides. Because of this repeating structure, probing the lambda EMBL3 E. tenella library with the insert subcloned into pBR325 will yield many unrelated phage that happen to contain the repeating structure. In order to obtain the entire gene for polypeptides containing the repeat, a different selection is required that makes use of synthetic oligonucleotides. This method has been used to obtain lambda ET::CHAD-19 which encodes polypeptide G. The method described below may also be used to isolate the entire gene for any of the polypeptides that contain the repeat.

Once it has been determined that a given lambda gt11 E. tenella expression clone contains the repeating CAG trinucleotide (by DNA cross-hybridization or by hybridization with a synthetic CAG containing oligonucleotide) the phage DNA is cleaved with EcoRI and the E. tenella insert is subcloned into the M13 mp8 or mp9 bacteriophage, described by Messing et al.in Gene 19:269-276 (1982), specifically incorporated herein by reference, and single stranded DNA is prepared from E. coli strain JM103 that has been transfected with the recombinant phage. This DNA serves as a template for the dideoxy DNA sequencing method developed by Sanger, F. et al. as described in PNAS(USA) 74:5463-5467 (1977), specifically incorporated herein by reference. Using standard procedures the DNA sequence for the E. tenella insert was obtained for CHAD-19. The sequence consists of the 255 nucleotides. Of the 85 residues encoded by the clone, there are 30 glutamine residues, and the CAG codon is used for 24 of the residues.

From the DNA sequence of the E. tenella insert two regions were selected that flank the repeating sequence and appear to be unique. For CHAD-19, nucleotides 163-195 [CH-1] and nucleotides 220-249 [CH-3] were selected. These regions were then checked against the published nucleotide squence of bacteriophage lambda to make sure that no significant regions of homology existed between the selected regions and the lambda EMBL3 cloning vector. Single-stranded DNA sequences corresponding to each of the two regions, CH-1 (33 nucleotides) and CH-3 (30 nucleotides), were then chemically synthesized using the solid phase system of Applied Biosystems Inc. The oligonucleotides were purified by polyacrylamide gel electrophoresis and end-labeled with $^{32}$P-ATP using polynucleotide kinase (P.L. Biochemicals).

The lambda EMBL3 E. tenella genomic library was then plated out on the Q359 E. coli strain at a density such that $4 \times 10^5$ phage plaques are present on 20 large petri plates. When the plaques were about 2 mm in diameter, they were lifted onto nitrocellulose filters using the method of Benton and Davis, in Science 196:180 (1977). Two replicas of each plate were made by sequential nicrocellulose lifts. The phage DNA that was transferred to the filters was denatured, neutralized, and baked. Each pair of the processed filters was then separately probed with each of the $^{32}$P-labeled synthetic oligonucleotides corresponding to the unique regions of the clone (CH-1 and CH-3) using stringent hybridization and wash conditions. The dry filters were exposed to X-ray film which was developed after a 24-hour exposure. Correct lamba EMBL3 E. tenella clones were identified by hybridization of both probes to the same plaque. These genomic phage were then purified, processed and mapped as described above. It is desired in these types of experiments that at least two probes be used in order to eliminated false-positives. By screening 4 x $10^5$ EMBL3::E. tenella, a genomic lambda ET::CHAD-19 clone that corresponds to the CHAD-19 DNA expression clone was obtained. A map of the lambda ET::CHAD-19 phage is contained in Fig. 4.

It will be apparent to those skilled in the art that various modifications and variations can be made in the processes of the present invention. Thus, it is intended that the present invention cover the modifications and variations of this invention provided that they come within the scope of the appended claims and their equivalents.

**Claims**

1. Protein H, a 25 kd protein of Eimeria tenella, produced by recombinant-DNA methods.

2. The phage lambda ET::Cal-2a.

3. A recombinant DNA method for the manufacture of polypeptides analogous to Eimeria protein H, which are capable of inducing an antigenic response, comprising:
   a. preparation of a DNA sequence capable of directing a host microorganism to produce a polypeptide possessing antigenic properties analogous to these possessed by a protein according to Claim 1;
   b. cloning the DNA sequence into a vector capable of being transferred into and replicating in a host microorganism, such vector containing operational elements for the DNA sequence;
   c. transferring the vector containing the DNA sequence and operational elements into a host microorganism capable of expressing the antigenic polypeptides;
   d. culturing the host microorganism under conditions appropriate for amplification of the vector and expression of the polypeptide; and
   e. in either order:
      (i) harvesting the polypeptide; and
      (ii) causing the polypeptide to assume a structure whereby it possesses antigenic properties analogous to properties possessed by polypeptides produced by Eimeria organisms.

# FIG. I.

λET::Rbl-8

LEFT ARM          RIGHT ARM

▓ = HYBRIDIZES TO cDNA

cDNA HYBRIDIZED TO
a) 5.5 kb, 1.k2 kb Sal = FRAGMENT
b) >23 kb RI FRAGMENT
c) >23 kb Hind III FRAGMENT
d) >23 kb Bam HI FRAGMENT

# FIG. 2.

λET::Rbl-17

LEFT ARM          RIGHT ARM

▓ = HYBRIDIZES TO cDNA
~15 kb INSERT

EP 0 614 973 A1

## FIG. 3.

λET : CH2-13

LEFT ARM

R R R R R

B B H B B

RIGHT ARM

S S S S H

= HYBRIDIZED TO cDNA
~155 kb INSERT

cDNA HYBRIDIZED TO
a) UNIQUE 3.5 kb EcoRI FRAGMENT
b) UNIQUE 7.5 kb SalI FRAGMENT
c) UNIQUE 12.5 kb Hind III FRAGMENT
d) 14.2 kb AND 2kb BamHI FRAGMENT

## FIG. 5.

λET :: Rb1-145

LEFT ARM

RI RI RI RI RI RI

RIGHT ARM

S B S H BS H

= HYBRIDIZED TO cDNA
~14 kb INSET
cDNA = 640bp

cDNA HYBRIDIZED TO
a) UNIQUE ~5.5 kb SalI FRAGMENT
b) ~1.8 + 1.5 kb EcoRI FRAGMENT
c) UNIQUE ~12.8 kb BamHI FRAGMENT
d) >23 kb Hind I FRAGMENT

EP 0 614 973 A1

# FIG. 4.

/CH2 - 15f    18,300 bp

NO Bam SITES

[███████████] = HYBRIDIZES TO cDNA

EP 0 614 973 A1

# FIG. 6.

λET :: CHAD-19

LEFT ARM    H    B    B    RIGHT ARM

S    S    S    H

▓▓▓▓ = HYBRIDIZES TO cDNA
~15kb INSERT

# FIG. 7.

λET : CAL-3gn

LEFT ARM    H    R H    HR    H    RIGHT ARM

S    S    S    B    S    H
B    B

▓▓▓ = HYBRIDIZED TO cDNA

cDNA HYBRIDIZED TO:
a) UNIQUE ~9.7kb Sal I FRAGMENT
b) UNIQUE ~3.6 kb Hind III FRAGMENT
c) UNIQUE ~1.3 kb BamHI FRAGMENT
d) UNIQUE ~13 kb Eco RI FRAGMENT

EP 0 614 973 A1

# FIG. 8.

λET∷Col−4cn

LEFT ARM   S                    E   E S S      S  E        E    E  E    S    RIGHT ARM

        B   B            B B BH      B B

        25          20          15          10          5    (kb)

✗ UNCERTAIN SITES

▨ = CROSSHYBRIDIZES TO cDNA

# FIG. 9.

MAP RBI−24b

PHASE λEmBL3/RBI−24b HAS THE cDNA HYBRIDIZATION
REGION IN THE MIDDLE AND IS OFFICIALLY MAPPED  A REPEAT
OF THE MAP IS BELOW                                    λET∶ RbI−24

                    λEMBL3/     ~18,000 bp
                    RBI−24b

                    H     H       H H           B       H

LEFT ARM     S      E     S     S          E S    S    RIGHT ARM

                    5000      10,000      15,000

▨ = HYBRIDIZES TO cDNA

## DOCUMENTS CONSIDERED TO BE RELEVANT

| Category | Citation of document with indication, where appropriate, of relevant passages | Relevant to claim | CLASSIFICATION OF THE APPLICATION (Int.Cl.4) |
|---|---|---|---|
| X | EP-A-0 164 176 (SOLVAY ET CIE) <br> * the whole document * <br> --- | 1-3 | C12N15/00 <br> G01N33/569 <br> C07K15/00 |
| X,P | EP-A-0 231 537 (SOLVAY ET CIE) <br> * the whole document * <br> --- | 1-3 | |
| X | WO-A-86 00528 (GENEX CORPORATION) <br> * the whole document * <br> --- | 1-3 | |
| E | EP-A-0 453 054 (SOLVAY ET CIE) <br> * the whole document * <br> --- | 1-3 | |
| E | EP-A-0 453 055 (SOLVAY ET CIE) <br> * the whole document * <br> ----- | 1-3 | |

TECHNICAL FIELDS
SEARCHED      (Int.Cl.4)

C12N
G01N
C07K

The present search report has been drawn up for all claims

| Place of search | Date of completion of the search | Examiner |
|---|---|---|
| THE HAGUE | 2 June 1994 | Skelly, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)